# EUROPEAN PATENT APPLICATION

(11) **EP 2 143 447 A1**
(43) Date of publication of application: **13.01.2010**
(21) Application number: 08012557.8
(22) Date of filing: 11.07.2008
(51) Int. Cl.: A61K 47/48, C12N 7/00, C12N 15/10, G01N 33/53

(54) **Bioconjugates comprising Aß-autoantibody-specific epitopes for active immunotherapy and diagnosis of Alzheimer's disease**

(71) Applicant: Universität Konstanz, 78464 Konstanz (DE)
(72) Inventor: Przybylski, Michael, 78465 Konstanz (DE); Manea, Marinela, 78467 Konstanz (DE); Hudecz, Ferenc, 1113 Budapest (HU); Mezo, Gabor, 1087 Budapest (HU)
(74) Representative: Müller-Boré & Partner Patentanwälte

(57) **Abstract**

Bioconjugates are provided comprising a scaffold and an antigen, wherein the antigen is a peptide comprising a region from the ß-amyloid peptide (Aß). Such bioconjugates are useful for immunotherapy and diagnosis of Alzheimer's disease.

## Description

### BACKGROUND

Alzheimer's disease (AD), the most common form of dementia among elderly populations (prevalence: 1000/100,000; > 65 years), represents the fourth leading cause of death in the developed world. Cortical atrophy, neuronal loss, region-specific amyloid deposition, neuritic plaques, and neurofibrillary tangles are key neuropathological features in AD brain. These alterations have been shown to be linked to cognitive decline which clinically defines AD. The degree of neuritic degeneration within plaques correlates with clinical parameters of dementia. Neuritic plaques are spherical, multicellular lesions that are usually found in moderate to large numbers in AD brain, and are comprised of extracellular deposits of amyloid-β peptide(s) (Aβ) as amyloid fibrils. The Aβ-plaques also contain variable numbers of activated microglia, often situated near the fibrillar amyloid core, as well as reactive astrocytes surrounding the core.

The major constituent of the neuritic plaque, β-amyloid peptide (Aβ), arises from a larger precursor protein, the amyloid precursor protein (APP). Aβ is produced by normal cells and can be detected in plasma and cerebrospinal fluid (CSF) of healthy individuals. Although the physiological role of APP in brain is still not well understood, missense mutations in APP have been shown to confer autosomal dominant inheritance of AD and have shed some light on the possible pathogenic mechanism(s). The accumulation of Aβ, a 39-42 amino acid proteolytic product of APP, in neuritic plaques has been established to be causative for AD progression. The major Aβ cleavage products of APP are the Aβ(1-42) peptide [SEQ ID NO.1], as well as Aβ- peptides shortened at the C-terminus (39 to 41 amino acids), which are produced by γ-secretase cleavage. The N-terminal part of Aβ(1-42) is localized in the extracellular region of APP, and the major C-terminal part of the Aβ peptide comprises a fragment of the transmembrane domain of APP.

Although APP has been recognised as a key molecule for AD, the molecular (patho-) physiological degradation and proteolytic pathways of APP, and cellular interactions and biochemical fate of Aβ peptide(s) are still unclear. Despite the lack of details on degradation pathways and cellular transport for the formation and deposition of Aβ-derived plaques, recent studies towards the development of immunisation methods of AD based on therapeutically active antibodies produced from Aβ(1-42) [SEQ ID NO.1] have yielded initial success in transgenic mouse models of Alzheimer's disease. Several reports have demonstrated that antibodies generated by active immunisation with Aβ(1-42) [SEQ ID NO.1] and corresponding Aβ-derived aggregates are capable of inhibiting the deposition of Aβ-plaques by disaggregating Aβ-fibrils, and improve the impairment in spatial memory of the mice. The transgenic APPV717F mouse (TG mouse) is a well characterized model of AD-like plaque pathology with age- and region-dependent deposits of Aβ(1-40) [SEQ ID NO.2] and Aβ(1-42) [SEQ ID NO.1] [Games et al. (1995), Alzheimer-type neuropathology in transgenic mice over expressing V717F beta-amyloid precursor protein. Nature 373: 523-527]. Recently, Schenk et al. and others investigated alterations in the deposition of Aβ in TG mice following immunization with pre-aggregated Aβ(1-42) or administration of antibodies against Aβ [Bard et al. (2000), Peripherally administered antibodies against amyloid beta-peptide enter the central nervous system and reduce pathology in a mouse model of Alzheimer disease. Nature Med 6: 916-919 and Schenk et al (1999), Immunization with amyloid-beta attenuates Alzheimer-disease-like pathology in the PDAPP mouse. Nature 400: 173-177].

Both active and passive immunization significantly attenuated amyloid plaque deposition, neuritic dystrophy, and astrogliosis. In these studies, increased titers of mouse anti-human Aβ-antibodies were observed for the reduction of plaques. These findings suggested that formation and clearance of Aβ-antigen:antibody complexes may decrease Aβ brain deposition either following antibody generation, or by peripheral antibody transport across the blood-brain-barrier. Furthermore, passive immunization appears to reduce brain Aβ burden by altering Aβ equilibrium between the CNS and plasma. Remarkably, active or passive immunization significantly reverses cognitive and memory impairment in TG or other APP transgenic mice. These results suggest that immunization may prevent memory deficits possibly by altering a soluble pool of Aβ. Thus, immunotherapy of AD patients using active or passive immunization is one of several emerging therapeutic approaches targeting the production, clearance, and aggregation of the Aβ peptide.

A first clinical trial using an active immunization procedure (Aβ(1-42) peptide [SEQ ID NO.1] and/or pre-aggregates thereof) was initiated for treatment of patients with established AD, but was stopped and discontinued due to toxicity related to brain inflammation effects observed in several patients ("meningoencephalitis"). However, it was demonstrated that (i), immunization induced the production of antibodies against Aβ and (ii), in patients where the production of antibodies was found, cognitive decline was significantly reduced in comparison to the untreated control group. It was concluded that immunization may exert an efficient therapeutic potential for AD.

Recent studies have addressed in detail the molecular recognition properties and Aβ-epitope specificity of antibodies produced upon immunisation with Aβ(1-42) [SEQ ID NO.1]. Selective proteolytic excision technologies of Aβ-antigens (epitope-excision) in combination with high resolution mass spectrometry resulted in the identification of a specific linear Aβ-epitope sequence recognised by the antibodies generated in transgenic AD mice, and in related Aβ-antibodies. The application of mass spectrometric epitope excision revealed the identification of the epitope to consist of the N-terminal residues (4-10) (FRHDSGY) of Aβ(1-42) [SEQ ID NO. 3]. The identical epitope was found in AD plaques, Aβ42 extracts from Aβ-protofibrils, chemically synthesised Aβ42, and other peptides comprising the N-terminal Aβ sequence [McLaurin et al. (2002), Therapeutically effective antibodies against amyloid-beta peptide target amyloid-beta residues 4-10 [SEQ ID NO. 3] and inhibit cytotoxicity and fibrillogenesis. Nature Med. 8, 1263-1269].

Recently, anti-Aβ- autoantibodies have been identified in both the blood and the CSF from non-immunized humans (healthy individuals). These antibodies specifically recognize human Aβ as shown by immunoprecipitation, ELISA and mass spectrometric characterization. The Aβ-autoantibodies readily recognize synthetic Aβ, as well as human Aβ in the brain of transgenic mice, and cause an inhibition and reduction of fibrillation and Aβ-related neurotoxicity. Remarkably, a substantially decreased Aβ-autoantibody concentration was found in patients with AD compared to healthy individuals. Aβ-autoantibodies have also been detected in commercially available IgG preparations (IV IgG), and treatment of AD patients with IV IgG led to the reduction of Aβ- concentrations in CSF. The neuroprotective effect of the Aβ-autoantibodies in inhibiting the deposition of Aβ-plaques was recently confirmed in first clinical studies, and led to a reduction of total Aβ upon administration of IV IgG. Since human anti-Aβ antibodies were found to block β-amyloid fibril formation and prevent β-amyloid-induced neurotoxicity, the application of these antibodies to AD patients can be expected to cause a reduction of Aβ concentration in CSF and to have a beneficial effect on cognitive functions.

Previously, there was general agreement that for an effective immunotherapeutic approach against established Aβ-plaques in brain, recognition and binding of an *N*-terminal epitope of Aβ, Aβ-derived peptides and aggregation products by antibodies is crucial. It is generally thought that recognition of the N-terminal Aβ sequence - which remains accessible to these therapeutically active antibodies - is aimed at plaque clearance.

By using epitope excision-mass spectrometry with a number of proteolytic enzymes of overlapping specificities [see e.g. Stefanescu R. et al. (2007), Mass spectrometric approaches to elucidation of antigen-antibody recognition structures in molecular immunology. Eur. J. Mass Spectrom. 13, 69-75], a new carboxyl terminal epitope, Aβ(21-37) (²¹AEDVGSNKGAIIGLMVG³⁷) [SEQ ID NO. 4], has been shown to be recognized by physiological Aβ-autoantibodies. These Aβ-autoantibodies were from healthy individuals (individual sera; adults of age range, 24-80; and pooled serum samples after affinity-isolation of Aβ-specific IgG). Deuterium exchange studies have further demonstrated the critical role of ³⁰AIIGLMVG³⁷ [SEQ ID NO. 5] to this recognition. See International Application No. PCT/IB2008/000456.

These results are in direct molecular contrast, and are complementary, to the recognition of the plaque-specific epitope of N-terminal Aβ, Aβ(4-10) [SEQ ID NO. 3], in antibodies obtained by active immunization with linear Aβ-peptides or a fibrillar Aβ-preparation. In all non-AD control sera, the carboxy-terminal Aβ(21-37) [SEQ ID NO. 4] sequence domain is specifically recognised. This epitope provides a conclusive molecular basis for the protective effect towards aggregate formation by neutralising antibodies. In contrast, the N-terminal epitope (Aβ4-10) [SEQ ID NO. 3] identical to that in transgenic AD mice was identified in antibodies from serum of AD patients, isolated by additional affinity chromatography. This N-terminal epitope domain previously has been shown to be independent of Aβ aggregation and fibril formation, and was shown to be identical to that recognised by monoclonal antibodies against N-terminal Aβ-peptides.

To generate plaque-protective Aβ-autoantibodies and to realize the potential of an immunotherapeutic approach to combating AD, effective strategies must be developed to effectively immunize patients with specific Aβ-epitopes.

### SUMMARY

According to a first aspect of this invention, there is provided a bioconjugate comprising a scaffold and an antigen associated therewith, wherein the antigen is a peptide comprising an amino acid sequence from a β-amyloid peptide consisting of EVHHQKLVFFAEDVGSNKGAIIGLMVG [SEQ ID NO. 23] or a fragment thereof comprising AIIGLMVG [SEQ ID NO. 5].

In another embodiment, a bioconjugate comprising a scaffold and an antigen associated therewith, wherein the antigen is a peptide comprising an amino acid sequence from a β-amyloid peptide, wherein said β-amyloid peptide sequence consists of EVHHQKLVFFAEDVGSNKGAIIGLMVG [SEQ ID NO. 23] or a fragment thereof comprising AIIGLMVG [SEQ ID NO. 5].

In another embodiment, a bioconjugate comprising a scaffold and an antigen associated therewith, wherein the antigen is a peptide comprising an amino acid sequence from a β-amyloid peptide comprising at least AIIGLMVG [SEQ ID NO. 5] and at most EVHHQKLVFFAEDVGSNKGAIIGLMVG [SEQ ID NO. 23].

In some embodiments, the antigen comprises peptides having the amino acid sequences of Aβ (30-37) [SEQ ID NO. 5], Aβ (29-37) [SEQ ID NO. 6]; Aβ (28-37) [SEQ ID NO. 7]; Aβ (27-37) [SEQ ID NO. 8]; Aβ (26-37) [SEQ ID NO. 9]; Aβ (25-37) [SEQ ID NO. 10]; Aβ (24-37) (SEQ ID NO. 11]; Aβ (23-37) [SEQ ID NO. 12], Aβ (22-37) [SEQ ID NO. 13]; Aβ (21-37) [SEQ ID NO 4]; Aβ (20-37) [SEQ ID NO 14]; Aβ (19-37) [SEQ ID NO 15]; Aβ (18-37) [SEQ ID NO 16]; Aβ (17-37) [SEQ ID NO 17]; Aβ (16-37) [SEQ ID NO 18]; Aβ (15-37) [SEQ ID NO 19]; Aβ (14-37) [SEQ ID NO 20]; Aβ (13-37) [SEQ ID NO 21]; Aβ (12-37) [SEQ ID NO 22]; and Aβ (11-37) [SEQ ID NO 23].

In some embodiments, the antigen can be a homo- or hetero-oligomer. In another embodiment, two or more antigens on one scaffold can associate to form a oligomer. In another embodiment, antigens on different scaffolds can associate to form an oligomer.

In other embodiments, the peptide can comprise additional amino acids N-terminal to the above sequences provided such amino acids do not include the sequence FRHDSGY [SEQ ID NO 3].

The inventive bioconjugates can utilize a variety of scaffolds. Exemplary scaffolds include, but are not limited to: virus like particles; liposomes; OT20 (oligotuftsin derivative), such as Ac-[TKPKG]₄-NH₂; SOC (sequential oligopeptide carrier), such as Ac-[Aib-Lys-Gly]₄-OH (Aib means 2 amnoisobutyric acid); MAP(multiple antigenic protein), such as H-KK(H-K)-RR-βA-NH₂; branched chain peptides and non peptides, such as poly[Lys(Serᵢ-DL-Alaₘ)], SAK poly[Lys(Gluᵢ-DL-Alaₘ)] and EAK.

In some embodiments, the scaffold of the bioconjugate is selected from the group consisting of an oligotuftsin derivative, a sequential oligopeptide carrier (SOC), a multiple antigenic protein (MAP), a branched peptide or non-peptide, virus like particles and liposomes.

In other aspects, oligotuftsin derivatives containing one or more (2-, 3- and 4-) chloroacetyl groups can be used as scaffolds. Examples of such oligotuftsin derivatives include:
Ac-[TKPK(ClAc-X)G][TKPKG][TKPKG][TKPKG]-NH₂
Ac-[TKPK(ClAc-X)G][TKPKG][TKPK(ClAc-X)G][TKPKG]-NH₂
Ac-[TKPK(ClAc-X)G][TKPK(ClAc-X)G][TKPK(ClAc-X)G][TKPKG]-NH₂
Ac-[TKPK(ClAc-X)G][TKPK(ClAc-X)G][TKPK(ClAc-X)G][TKPK(ClAc-X)G]-NH₂ wherein
X is a chemical bond, GFLG or GGGGG.

In one embodiment, the bioconjugate has the formula: 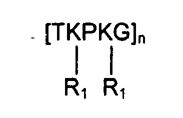 wherein T is threonine, K is lysine, P is proline, G is glycine and R₁ is a residue representing either H (hydrogen) or -X-Z, wherein X is a linker, wherein Z is a dimer of the peptide with the proviso that at least one R₁ is not H, and n is an integer from 2 to about 50, preferably from 2 to about 20. In some embodiments, n is 2 to 10, while in others it is 2 to 4.

In some embodiments, the linker is selected from the group consisting of a thioether linkage, a polyglycine residue having 4-12 glycine residues, a short peptide having the amino acid sequence GFLG and an oligoethylene glycol.

In another aspect, a pharmaceutical composition is provided that comprises a bioconjugate and a T-helper cell epitope, where the bioconjugate comprises a scaffold and an antigen associated therewith, wherein the antigen is a peptide comprising an amino acid sequence from a β-amyloid peptide comprising at least AIIGLMVG [SEQ ID NO. 5] and at most EVHHQKLVFFAEDVGSNKGAIIGLMVG [SEQ ID NO. 23]. The T-helper cell epitope can be in the form of an isolated peptide. Alternatively, the T-helper cell epitope is contained within a polypeptide that is associated with the bioconjugate.

In another aspect, a vaccine is provided that comprises a pharmaceutical composition comprising a bioconjugate and a T-helper cell epitope, where the bioconjugate comprises a scaffold and an antigen associated therewith, wherein the antigen is a peptide comprising an amino acid sequence from a β-amyloid peptide comprising at least AIIGLMVG [SEQ ID NO. 5] and at most EVHHQKLVFFAEDVGSNKGAIIGLMVG [SEQ ID NO. 23].

In another embodiment, methods for the *in vitro* diagnosis of Alzheimer's disease in a patient are provided that comprise reacting a patient sample with a bioconjugate of the present invention and detecting the binding of specific antibodies to the bioconjugate.

Other objects, features and advantages will become apparent from the following detailed description. The detailed description and specific examples are given for illustration only since various changes and modifications within the spirit and scope of the invention will become apparent to those skilled in the art from this detailed description. Further, the examples demonstrate the principle of the invention and cannot be expected to specifically illustrate the application of this invention to all the examples where it will be obviously useful to those skilled in the prior art.

### BRIEF DESCRIPTION OF THE FIGURES

### Figure 1

Immunotherapeutic principles in Alzheimer's disease by Aβ-plaque-disaggregating and inhibiting antibodies. Therapeutic strategies aim to prevent and reverse the pathological events leading to amyloid plaque formation: modulation of Aβ production; inhibition of Aβ aggregation and stimulation of the clearance of soluble Aβ and amyloid deposits from the brain by Aβ reactive antibodies.

### Figure 2

Identification of Aβ(21-37) [SEQ ID NO. 4] as an epitope in Aβ(1-40) [SEQ ID NO. 2] peptide recognized by Aβ-autoantibodies. Sequences of the eluted fragments from epitope excision/extraction MS experiments with GluC and pronase are underlined; the overlapping of the proteolytic fragments by different proteases is indicated by solid lines.

### Figure 3

Proposed mechanism of action of "plaque-specific" and "plaque-protective" antibodies. "Plaque-specific" antibodies bind to the N-terminal Aβ(4-10) [SEQ ID NO. 3] domain which is accessible in amyloid fibrils. Plaque breakdown by activated microglia through Fc receptor mechanisms could explain the Aβ plaque clearance effect of these antibodies. "Plaque-protective" antibodies recognize the C-terminal epitope Aβ(21-37) [SEQ ID NO. 4] leading to the inhibition of Aβ- aggregation, by sequestering the soluble Aβ from blood, and efflux of Aβ from brain to the CSF.

### Figure 4

RP-HPLC purification and MALDI-FT-ICR mass spectrum of synthetic biotin-G₅-Aβ(12-40) [SEQ ID NO. 24] peptide. Solid-phase synthesis of biotin-G₅-Aβ(12-40) was performed with a semiautomated peptide synthesizer.

Details of synthesis, HPLC purification and mass spectrometric characterization are described below.

### Figure 5

Schematic representation of the isolation and purification protocol of Aβ-autoantibodies from human serum using an antigen-affinity column consisting of Cys-Aβ(12-40) [SEQ ID NO. 24] attached *via* a thioether bond to Ultralink-iodoacetyl-Gel. The IgG preparation or human serum are incubated overnight with the affinity column; unbound IgG material is then removed by washing, followed by the specific elution of Aβ- antibodies.
Positive control for the column was performed using ELISA (Enzyme Linked Immunosorbent Assay) using the following protocol:
1. Coat a 96-well microtiter plate with 100 µl/well of µM Aβ1-40 [SEQ ID NO. 2] in PBS (pH 7,5) overnight at 4°C
2. Discard the solution and wash 4x15 min with 200µl/well PBS-Tween (pH 7,5)
3. Block remaining active sites with 200µl/well 5%BSA in PBS-Tween 3h at room temperature
4. Discard the solution and wash 4x15 min with 200µl/well PBS-Tween (pH 7,5)
5. Dilute anti Aµ12-40 antibody separated with the column from 1:10 µg/µl to 1:21870 (dilution 1:3 for each step) in blocking buffer. Incubate 2h at room temperature
6. Discard the solution and wash 4x15 min with 200 ml/well PBS-Tween (pH 7,5)
7. Dilute second antibody 1:5000 (µg/µl) in blocking buffer and incubate 2h at room temperature
8. Discard the solution and wash 4x15 min with 200µl/well PBS-Tween (pH 7,5)
9. Discard the solution and wash 1x15 min with 200µl/well citrate phosphate (pH 5)
10. Add 100µl/well substrate solution (1mgODP/ml citrate solution, 2µl H₂O₂/10 ml citrate solution)
11. Read absorbance at 450nm in Wallac ELISA plate reader.

### Figure 6

ELISA affinity determination of epitope-specific Aβ-autoantibodies in IgG preparation (left) and human sera from healthy individuals (right) to biotinylated G₅-Aβ(1-40) [SEQ ID NO. 2] peptide, illustrating the presence of Aβ-specific antibodies in IgG and human serum.

### Figure 7

General scheme for the design of synthetic peptide based vaccine conjugates by incorporation of multiple copies of a B-cell epitope and a promiscuous helper T-cell epitope in a polymer-carrier architecture.

### Figure 8

Schematic structure representation of bioconjugates in which Aβ(4-10) [SEQ ID NO. 3] or T-helper cell-Aβ(4-10) epitope peptides were attached through a thioether bond to branched chain polymeric peptide carriers, SAK and EAK. Polymeric carriers are based on a poly[L-Lys] backbone and contain short side chains composed of 3 DL-Ala residues, and Ser or Glu residues at the end of the branches.

### Figure 9

Binding of mouse anti-Aβ(1-17) monoclonal antibody to Aβ(4-10) [SEQ ID NO. 3] epitope peptide conjugates and to T-helper cell-Aβ(4-10) epitope peptide, determined by ELISA. The antigenic properties of bioconjugates were influenced by the carrier structure and epitope topology.

### Figure 10

Amino acid sequences of synthetic β-amyloid peptides used for binding studies to Aβ-autoantibodies. All peptides were elongated by a pentaglycine spacer and biotinylated at the N-terminus. The syntheses were performed on a NovaSyn TGR resin according to the Fmoc/tBu strategy. Crude peptides were purified by RP-HPLC and analyzed by mass spectrometry as described.

### Figure 11

Procedure used for the synthesis of an oligotuftsin polypeptide carrier functionalized by Lys-chloroacetylation.

The chloroacetylated tuftsin carrier were synthesized by using the Boc/Bzl strategy as previously published [DeMattos RB et al. (2001), Peripheral anti-A beta antibody alters CNS and plasma A beta clearance and decreases brain A beta burden in a mouse model of Alzheimer's disease. Proc Natl Acad Sci USA 98: 8850-8855]. Tetratuftsin derivatives with or without a tetrapeptide GFLG spacer in the branches (Ac-[TKPK(ClAc)G]₄-NH₂, and Ac-[TKPK(ClAc-GFLG)G]₄-NH₂,) were prepared on MBHA resin (1.04 mmol/g). The chloroacetylated MAP construct (ClAc-K(ClAc)-K(ClAc-K(ClAc))RRβA-NH₂) was built up also on MBHA resin, with a lower coupling capacity (0.67 mmol/g), while the SOC derivative (Ac-[K(ClAc)AibG]₄-OH) was assembled on a PAM resin (0.72 mmol/g) (19). A chloroacetylated tetratuftsin derivative analogue, elongated at the N-terminus with a promiscuous helper T-cell epitope derived from a hepatitis B surface antigen (Ac-FFLLTRILTIPQSLD-[TKPK(ClAc)G]₄-NH₂) (not shown) was also synthesized on MBHA resin (0.67 mmol/g capacity). The following side chain protected N^{ε}-Boc-amino acid derivatives were used: Boc-Arg(Tos)-OH, Boc-Asp(OcHex)-OH, Boc-Lys(CIZ)-OH, Boc-Lys(Fmoc)-OH, Boc-Ser(Bzl)-OH, Boc-Thr(Bzl)-OH. The protocol of the synthesis was as follows: (i) DCM washing (3x0.5 min), (ii) Boc cleavage using 33% TFA in DCM (2+20 min), (iii) DCM washing (5x0.5 min), (iv) neutralization with 10% DIEA in DCM (4x1 min), (v) DCM washing (4x0.5 min), (vi) coupling 3 equivalents of Boc-amino acid derivative-DCC-HOBt in DCM-DMF mixture (4:1 or 1:4 v/v depending on the solubility of the amino acid derivatives) (60 min), (vii) DMF washing (2x0.5 min), (viii) DCM washing (2x0.5 min), (ix) ninhydrine or isatin assay to assess the presence of free amines or imines. After completion of the synthesis, the acetylation of the N-terminus was carried out with acetic anhydride (1 mL) and DIEA (1 mL) in DMF (3 mL) for 1 hr at room temperature. Prior to the chloroacetylation of the appropriate Lys side chains with chloroacetic acid pentachlorophenyl ester (ClAcOPcp), the Fmoc protecting groups were removed with 2 % DBU-2 % piperidine in DMF (four times for 2+2+5+10 min). The peptide was cleaved from the resin simultaneously with the removal of the side-chain protecting groups using liquid HF (10 mL) in the presence of *m*-cresol (0.5 mL) and *p*-thiocresol (0.5 g) as scavangers (20). The crude product was purified by RP-HPLC on a semi-preparative C₄ column, and structural integrity was verified by MALDI-MS.

### Figure 12

This figure provides a structural model of an oligotuftsin polypeptide.

### Figure 13 a, b

Structure model of oligotuftsin polypeptide T20 (**a**) and accessibilities of lysine residues (**b**). The structure model represents the simulation from the lowest energy structure. The black colors show the Lys side chains. Calculations of average relative solvent accessibilities for the eight Lys ε-amino groups were performed by taking the average accessibility of the complete molecule as 100 %.

### Figure 14

General scheme for the synthesis of oligomeric tuftsin-Aβ-epitope conjugates by chemoselective ligation, using thioether bond formation of cysteine containing β-amyloid peptides to chloroacetylated oligotuftsin derivative carriers.

### Figure 15

HPLC- Purified tri/tetrameric oligotuftsin-Cys-Aβ(20-37) conjugate ("4-copy-Aβ-epitope conjugate") by conjugation with a chloroacetylated oligotuftsin derivative carrier (Ac-[T20(ClAc-GFLG)₄]), analyzed by MALDI-MS at 1 h (a), 2 hrs (b), 24 hrs (c), and HPLC-purified conjugate at 24 hrs (d).

HPLC purification was performed on a Bio-Rad system (Bio-Rad Laboratories, Richmond, CA) using either Nucleosil 300-7 C₁₈ (250x4 mm, 300 Å, 7 µm) (Macherey-Nagel, Düren, Germany) or Grom Protein-III C₄ (250x4.6 mm I.D.) with 7 µm silica (300 Å pore size) (Herrenberg-Kayh, Germany) columns. Linear gradient elution (0 min 0% B; 5 min 0% B; 55 min 100% B) with eluent A (0.1% TFA in water) and eluent B (0.1 % TFA in acetonitrile-water (80:20 v/v)) was used at a flow rate of 1 mL/min, and samples were dissolved in eluent A. Semipreparative HPLC purification was performed on a semi-preparative Phenomenex Jupiter C₁₈ column (250x10 mm I.D.) with 10 µm silica (300A pore size) (Torrance, CA) as a stationary phase. Linear gradient elution (0 min 10% B; 5 min 10% B; 45 min 50% B) was used in all cases except Ac-T20 derivatives) with eluent A (0.1 % TFA in water) and eluent B (0.1 % TFA in acetonitrile-water (80:20, v/v)). Peaks were detected at 220 nm.

MALDI-MS was performed on a Bruker Biflexll MALDI-TOF spectrometer (Bruker Daltonoics, Bremen, Germany), and conditions employed as described previously.

### Figure 16

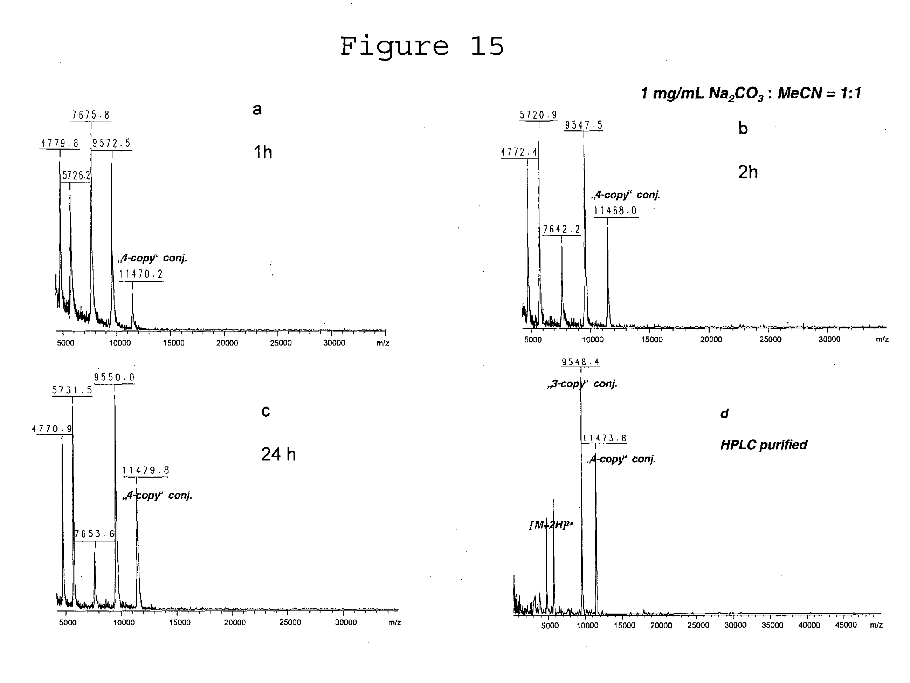

Tetrameric oligotuftsin-Cys-Aβ(20-30) conjugate of a chloroacetylated oligotuftsin derivative carrier (Ac-[T20(ClAc-GFLG)₄]) with Ac-Cys-Aβ(20-30) peptide [SEQ ID NO. 26], analyzed by MALDI-MS. Conditions for HPLC purification and MALDI-MS were as described in Figure 15.

### Figure 17

Binding affinities of human IgG (Calbiochem) to Aβ(1-40) [SEQ ID NO. 2] and oligotuftsin-Aβ-epitope bioconjugates determined by ELISA. Aβ-autoantibodies are shown to exert binding specifically to Aβ(1-40) [SEQ ID NO. 2] and the Aβ(20-37)-epitope-oligotuftsin conjugate, but not to oligotuftsin conjugates containing the Aβ(20-30) peptide [SEQ ID NO. 26], or to the oligotuftsin carrier.

ELISA binding studies were performed with the following general protocol:
1. coating the 96-well ELISA plates with 100 µL/well of conjugate solution (c=1 or 5 µM in PBS, pH 7.4); overnight incubation at room temperature.
2. washing the plates four times with 200 µL/well of washing buffer (PBS-T: 0.05% Tween-20 in PBS buffer, pH 7.4).
3. blocking with 200 µL/well of blocking buffer (5% BSA, 0.1% Tween-20 in PBS), overnight at 4°C.
4. washing once with 200 µL/well PBS-T.
5. 100 vL/well of isolated Aβ-autoantibody or human immunoglobulin preparation (Calbiochem) were added at an initial dilution of 1:100, then diluted 3 fold serially in sample dilution buffer (5% BSA, 0.1 % Tween-20, 1 % DMSO in PBS) and incubated for 2 hrs at room temperature.
6. washing six times with PBS-T.
7. 100 vL/well of goat anti-human IgG conjugated with horseradish peroxidase diluted 1:5000 in blocking buffer are added to the plates and incubated for 1 h at room temperature.
8. washing three times with 200 µL/well of PBS-T and once with 200 µL/well of 0.05 M sodium phosphate-citrate buffer, pH=5.
9. 100 µL of *o*-phenylenediamine dihydrochloride (OPD) in substrate buffer (phosphate-citrate) at c=1 mg/mL with 2 µL of 30% hydrogen-peroxide per 10 mL of substrate buffer are added.
10. The absorbance at 450 nm is measured on a Wallac 1420 Victor² ELISA Plate.

### Figure 18

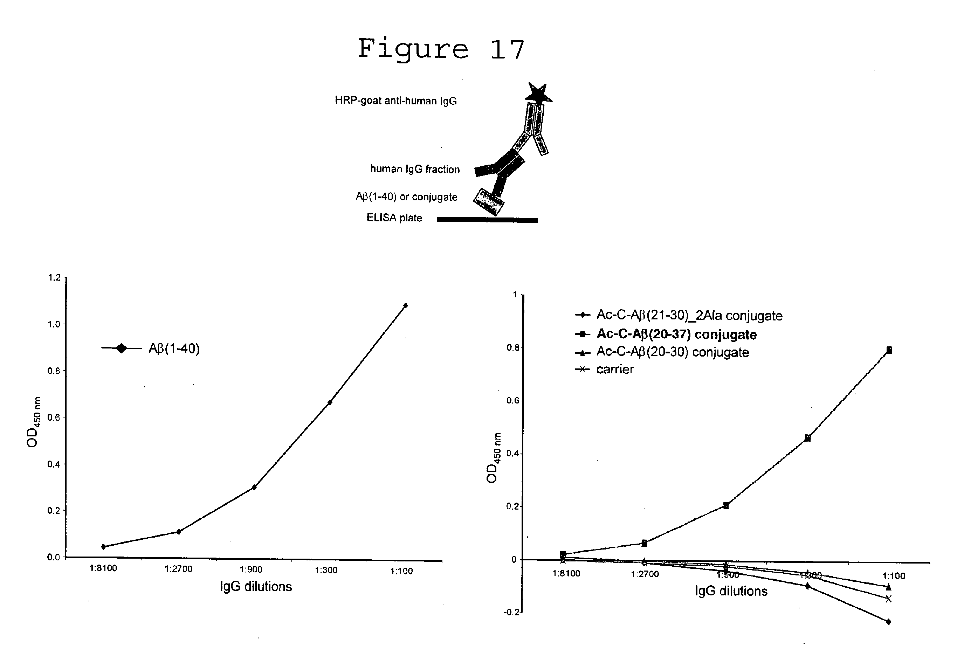

Binding affinities of affinity-purified Aβ-autoantibodies to Aβ(1-40) [SEQ ID NO. 2] and oligotuftsin-Aβ-epitope bioconjugates determined by ELISA. Aβ-autoantibodies are shown to exert binding specifically to the Aβ(20-37)-epitope-oligotuftsin conjugate, but not to oligotuftsin conjugates containing the Aβ(20-30) peptide [SEQ ID NO. 26], or to the oligotuftsin carrier. Conditions for ELISA were identical to those described in Figure 17.

### Figure 19

MALDI-MS analysis of soluble Aβ-oligomers from Aβ(1-40) [SEQ ID NO. 2] separated by SDS-gel electrophoresis as a approximately 16 kDa band (MALDI-TOF-MS).

### Figure 20

Model structure of β-amyloid fibrils within the core structure of the Aβ(17-42) sequence [SEQ ID NO. 28], determined by a combination of NMR and molecular simulation. Aβ-fibrils are shown to comprise two parallel β-strands formed by residues (18-26) and (31-42), separated by a 180° bend within the residues (27-30).

### Figure 21(a-c)

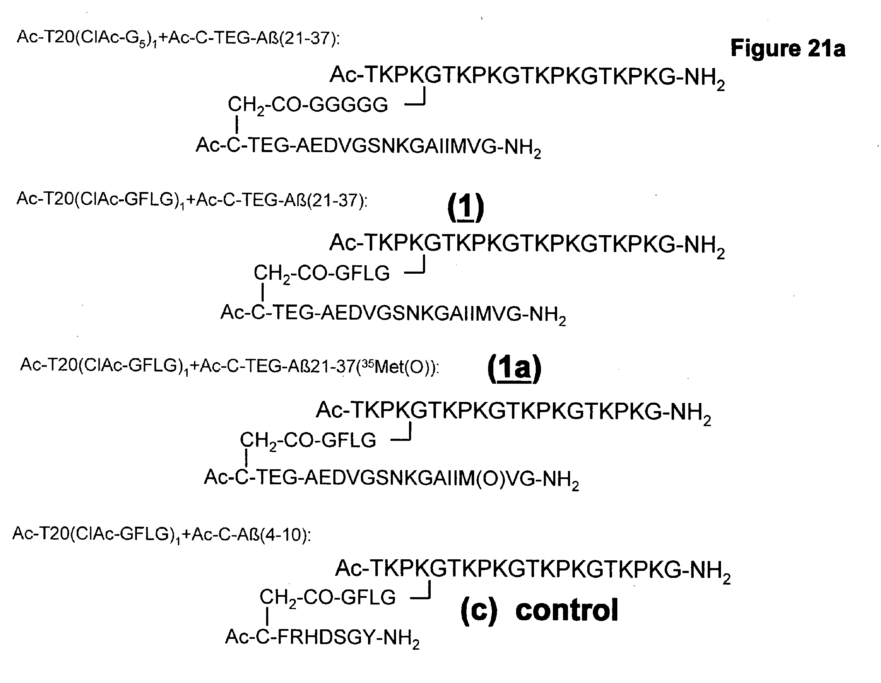

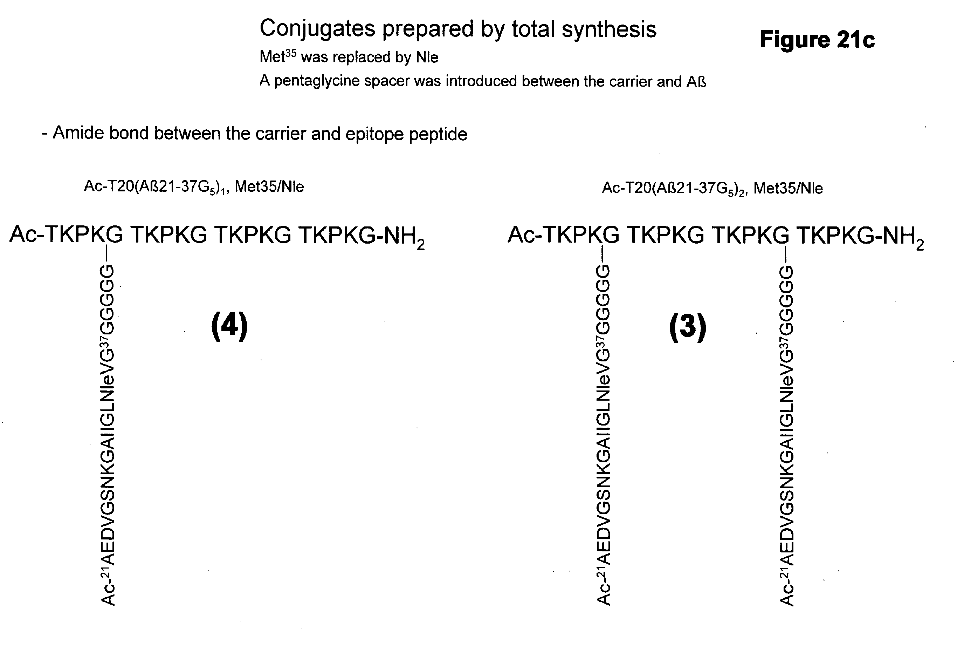

Structures of "monomeric" and "dimeric" oligotuftsin-Aβ (21-37) conjugates with oligopeptide -GFLG-, tetraethylene glycol (TEG), and pentaglycine spacer groups (**1, 1a - 4; cf.** **Figure 21a****-c).** Polypeptides were synthesized as described and conjugated via N-terminal cysteine residues. Conjugates with pentaglycine containing 1 and 2 copies of Aβ-epitope peptides attached at the C-terminal G37-residue (**Figure 21c**) were prepared by total synthesis. Met-35 Residues were replaced by norleucine as indicated. The conjugate **(c)** of N-terminal Aβ(4-10) epitope , used as a control was synthesized with identical structure.

### Figure 22

Binding affinities of human IgG (Calbiochem) to oligotuftsin-Aβ(21-37)-epitope bioconjugates containing 1 and/or 2 copies of Aβ(21-37) [SEQ ID NO. 4] determined by ELISA. The oligotuftsin-conjugates with a single copy of Aβ(4-10) epitope was used as a control. While not shown, Aβ(1-40) [SEQ ID NO. 2] was used as a positive control, as shown in Figure 17. ELISA Determinations were carried out as described in Figure 17.

### Figure 23a-e

The structure of several constructs according to the present invention is shown. The conjugates are branched structures whereby one or more epitopes are linked to the scaffold molecule.

New approaches for diagnosing and treating neurodegenerative diseases related to AD are provided. In this regard, bioconjugates are provided that comprise a scaffold and an antigen, wherein the antigen is a peptide comprising a region from the β-amyloid peptide (Aβ). Immuno-therapy employing such bioconjugates induce protective antibodies with a specificity related to that of physiological Aβ-autoantibodies.

In one aspect, a bioconjugate comprising a scaffold and an antigen associated therewith, wherein the antigen is a peptide comprising an amino acid sequence from a β-amyloid peptide consisting of EVHHQKLVFFAEDVGSNKGAIIGLMVG [SEQ ID NO. 23] or a fragment thereof comprising AIIGLMVG [SEQ ID NO. 5]. In another apsect, a bioconjugate comprises a scaffold and an antigen associated therewith, wherein the antigen is a peptide comprising an amino acid sequence from a β-amyloid peptide comprising at least AIIGLMVG [SEQ ID NO. 5] and at most EVHHQKLVFFAEDVGSNKGAIIGLMVG [SEQ ID NO. 23].

A "scaffold" denotes any structure upon which peptides can be attached or incorporated in a stable manner. This structure serves as a backbone of the bioconjugate. Exemplary scaffolds include, but are not limited to:
- Virus like particles
- liposomes
- OT20 (oligotuftsin derivative): Ac-[TKPKG]₄-NH₂
- SOC (sequential oligopeptide carrier) Ac-[Aib-Lys-Gly]₄-OH (Aib means 2 aminoisobutyric acid)
- MAP(multiple antigenic protein) H-KK(H-K)-RR-βA-NH₂
- Branched chain peptides and non peptides such as poly[Lys(Serᵢ-DL-Alaₘ)]; SAK and poly[Lys(Gluᵢ-DL-Alaₘ)]; EAK

The antigen of the bioconjugate may be in monomeric or oligomeric form at the time of first association with the scaffold. If in monomeric form, it is expected that oligomerisation will occur by interaction between adjacent monomers on the same or different scaffolds.

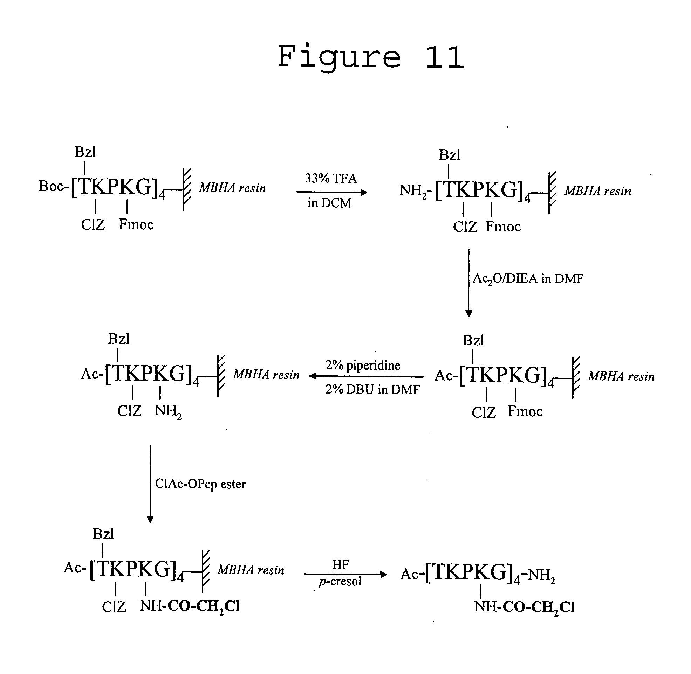

In one aspect, an oligotuftsin derivative is used as a scaffold. Tuftsin is a sequential oligopeptide consisting of four tandem repeat units of a pentapeptide (Ac-[TKPKG]₄-NH₂, Ac-T20) derived from the canine tuftsin. *See*, *e*.*g*. Mezo et al., J. Peptide Science, 12(5):328-336 (2005). Oligotuftsins such as the tetratuftsin derivative T20 (**Figures 11-14**) are non-toxic, non-immunogenic and exhibit tuftsin like biological properties, e.g. immunostimulatory activity and chemotactic activity. In a preferred embodiment, an oligotuftsin derivative is derived from the canine tuftsin sequence (TKPK), which differs from human tuftsin only in the C-terminal amino acid, where arginine has been replaced by lysine.

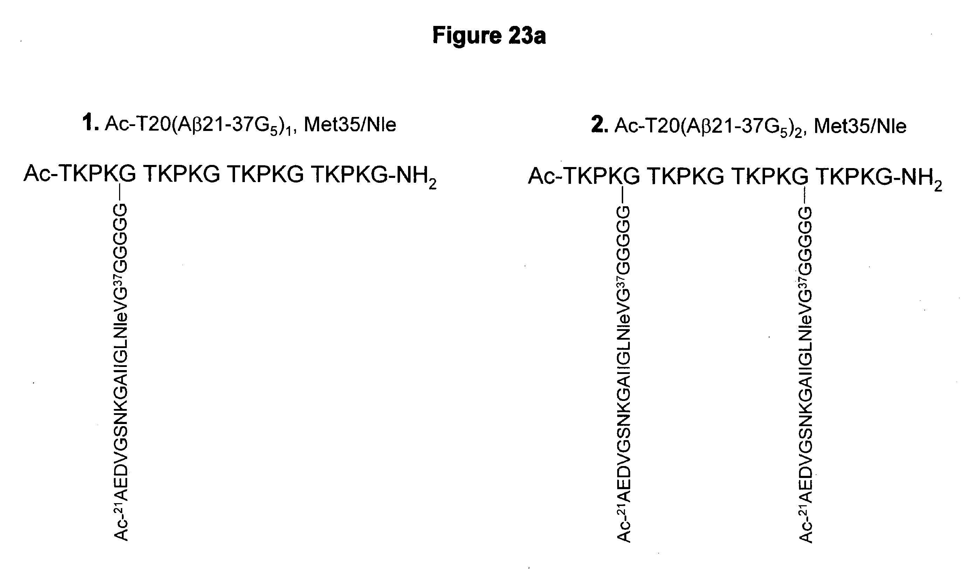

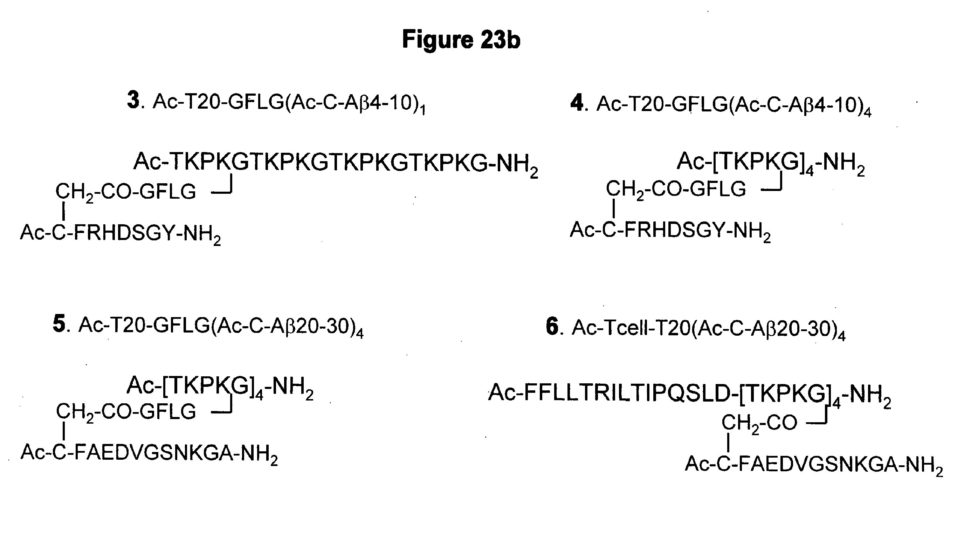

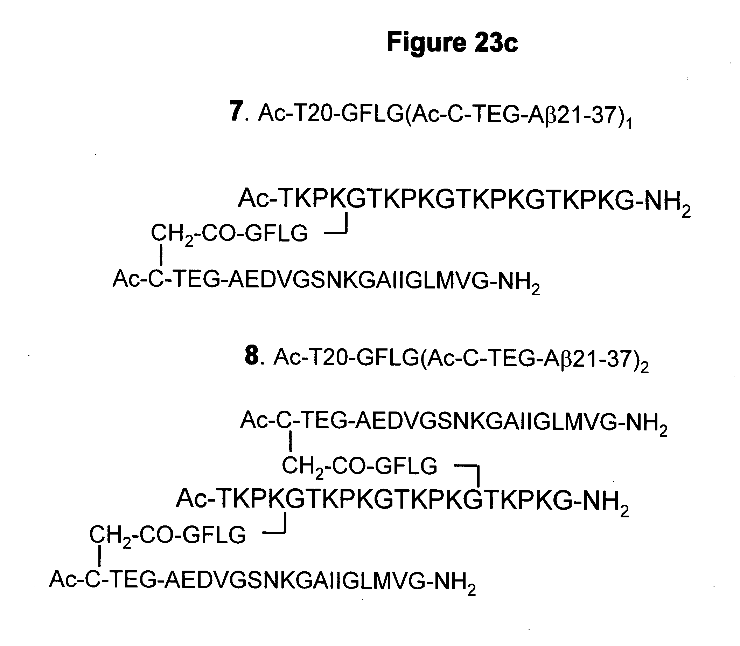

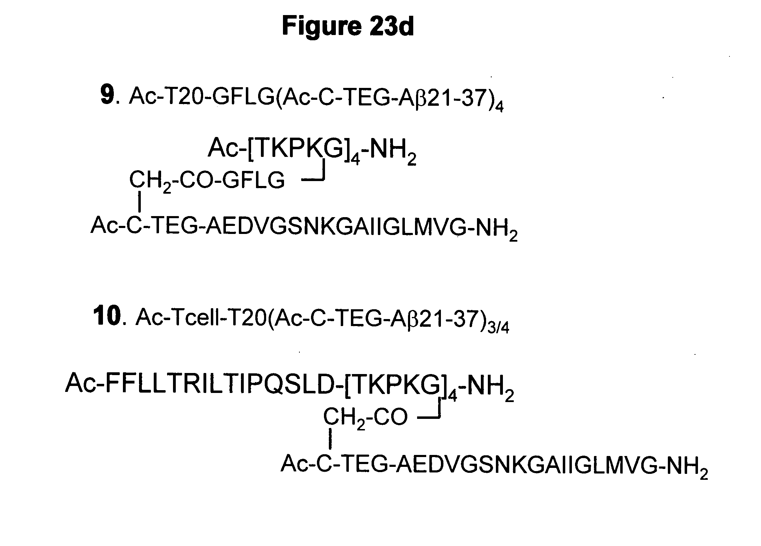

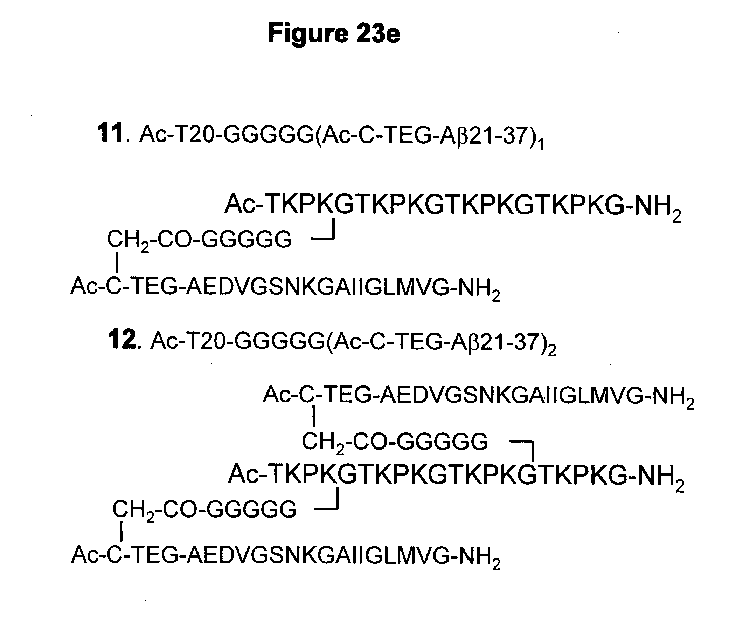

In some embodiments, a oligotuftsin derivative comprises the formula [TKPKG]ₙ where n = 4 to about 50. Exemplary bioconjugates employing such oligotuftsin derivatives are shown in Fig. 23a-e.

Such bioconjugates can be associated with one or more Aβ- epitopes and can:
(i) bind and isolate Aβ-oligomer specific antibodies with high efficiency and sensitivity;
(ii) serve as specific scaffolds for the detection and diagnostic determination of oligomer/polymer-specific Aβ-antibodies;
(iii) serve as lead structures for the design and preparation of new Aβ-oligomer specific immunogens and vaccine conjugates, for immunotherapy of AD and AD-related diseases. Such immunotherapy may be directed to the prevention of disease or, where disease already exists, such immunotherapy may be used to slow down, halt or even reverse the symptoms of disease.

In other embodiments, oligotuftsin derivatives containing one or more (2-, 3- and 4-) chloroacetyl groups can be used. Exemplary oligotuftsin derivatives include:
Ac-[TKPK(ClAc-X)G][TKPKG][TKPKG][TKPKG]-NH₂
Ac-[TKPK(ClAc-X)G][TKPKG][TKPK(CIlc-X)G][TKPKG]-NH₂
Ac-[TKPK(ClAc-X)G][TKPK(ClAc-X)G][TKPK(ClAc-X)G][TKPKG]-NH₂
Ac-[TKPK(ClAc-X)G][TKPK(ClAc-X)G][TKPK(ClAc-X)G][TKPK(ClAc-X)G]-NH₂ wherein
X is a chemical bond, GFLG or GGGGG.

In further embodiments, oligotuftsin derivatives possess the formula: 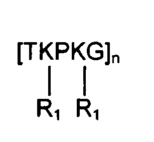 wherein T is threonin, K is lysine, P is proline, G is glycine and R₁ is a residue representing either H (hydrogen) or -X-Z; wherein X is a linker; and wherein Z is a polypeptide having the amino acid sequences Aβ (30-37) [SEQ ID NO. 5], Aβ (29-37) [SEQ ID NO. 6]; Aβ (28-37) [SEQ ID NO. 7]; Aβ (27-37) [SEQ ID NO. 8]; Aβ (26-37) [SEQ ID NO. 9]; Aβ (25-37) [SEQ ID NO. 10]; Aβ (24-37) (SEQ ID NO. 11]; Aβ (23-37) [SEQ ID NO. 12], Aβ (22-37) [SEQ ID NO. 13); Aβ (21-37) [SEQ ID NO 4]; Aβ (20-37) [SEQ ID NO 14]; Aβ (19-37) [SEQ ID NO 15); Aβ (18-37) [SEQ ID NO 16]; Aβ (17-37) [SEQ ID NO 17]; Aβ (16-37) [SEQ ID NO 18]; Aβ (15-37) [SEQ ID NO 19]; Aβ (14-37) [SEQ ID NO 20]; Aβ (13-37) [SEQ ID NO 21]; Aβ (12-37) [SEQ ID NO 22]; or Aβ (11-37) [SEQ ID NO 23]; and n is an integer from 2 to about 50, preferably from 2 to about 20. In some embodiments, n is 2-10 or 2-4.

The antigen of the bioconjugates is a peptide comprising an amino acid sequence from a β-amyloid peptide comprising at least AIIGLMVG [SEQ ID NO. 5] and at most EVHHQKLVFFAEDVGSNKGAIIGLMVG [SEQ ID NO. 23]. Exemplary antigens include, but are not limited to peptides having the amino acid sequences of Aβ (30-37) [SEQ ID NO. 5], Aβ (29-37) [SEQ ID NO. 6]; Aβ (28-37) [SEQ ID NO. 7]; Aβ (27-37) [SEQ ID NO. 8]; Aβ (26-37) [SEQ ID NO. 9]; Aβ (25-37) [SEQ ID NO. 10]; Aβ (24-37) (SEQ ID NO. 11]; Aβ (23-37) [SEQ ID NO. 12], Aβ (22-37) [SEQ ID NO. 13]; Aβ (21-37) [SEQ ID NO 4]; Aβ (20-37) [SEQ ID NO 14]; Aβ (19-37) [SEQ ID NO 15]; Aβ (18-37) [SEQ ID NO 16]; Aβ (17-37) [SEQ ID NO 17]; Aβ (16-37) [SEQ ID NO 18]; Aβ (15-37) [SEQ ID NO 19]; Aβ (14-37) [SEQ ID NO 20]; Aβ (13-37) [SEQ ID NO 21]; Aβ (12-37) [SEQ ID NO 22]; and Aβ (11-37) [SEQ ID NO 23].

In some embodiments, the antigen is an oligomer. The terms "oligomer" and "oligomeric" denote two or more peptides in association. Homo-oligomers comprise multiple copies of one peptide, while hetero-oligomers comprise different peptides. In one embodiment, the oligomer is a tetramer (i.e., a double dimer).

In some embodiments the antigen may comprise additional amino acids N-terminal to the above sequences provided such amino acids do not include the sequence FRHDSGY [SEQ ID NO. 3], whereby said sequences are present as oligomers or dimers with the proviso that with respect to the oligotuftsin derivative formula above at least one residue R₁ is not hydrogen. The linker X may be a chemical bond and/or a short peptide having 2 to 20 amino acids. A preferred linker is -GGGGG-.

In other embodiments, the bioconjugate also can comprise a T-helper cell antigen. Exemplary T-cell antigens include the amino acid sequences disclosed in Table 1.

**Table 1.**

| Source/protein | Amino acids | T-helper cell epitope sequence | SEQ ID NO: |
|---|---|---|---|
| Malaria/circumsporozoite of *P. falciparum* | 380-398 | EKKIAKMEKASSVFNVVNS | 29 |
| | 331-350 | IEQYLKKIKNSISTEWSPCS | 30 |
| *Clostridium tetani*/tetanus toxoid | 830-844 | QYIKANSKFIGITEL | 31 |
| | 947-967 | FNNFTVSFWLRVPKVSASHLE | 32 |
| | 580-599 | NSVDDALINSTKIYSYFPSV | 33 |
| | 916-932 | PGINGKAIHLVNNESSE | 34 |
| | 830-843 | QYIKANSKFIGITE | 35 |
| Influenza virus/hemagglutinin | 17-29 | SGPLKAEIAQRLE | 36 |
| | 307-319 | PKYVKQNTLKLAT | 37 |
| Measles virus/fusion protein | 288-302 | LSEIKGVIVHRLEGV | 38 |
| Hepatitis B virus/HBsAg | 19-33 | FFLLTRILTIPQSLD | 39 |
| Hepatitis A virus/VP1 | 17-33 | NVPDPQVGITTMRDLKG | 40 |
| | 276-298 | MSRIAAGDLESSVDDPRSEEDR R | 41 |
| Papillomavirus/E7 | 48-54 | DRAHYNI | 42 |

The epitopes may exist as isolated T-helper cell epitopes such as those identified in Table 1. These epitopes will preferably be covalently attached to the same scaffold to which the antigen is attached. Alternatively, the epitopes may be contained within a larger protein molecule. The molecule will preferably be covalently attached to the scaffold or otherwise associated therewith.

The antigen of the bioconjugate is associated with the scaffold. In this regard, the nature of the association is that the antigen is attached or conjugated to the scaffold. In one embodiment, the antigen is covalently linked to the scaffold. In another embodiment, the antigen is attached via non-covalent interactions, such as hydrophobic interactions, ionic bonding, hydrogen bonding, Van der Waals force and dipole-dipole bonds. In another embodiment, the antigen is attached to the scaffold via a linker.

Any suitable linker known in the field can be employed. Such linkers should not induce undesired immunological side-reactions but provide a covalent bond between the scaffold and the antigen. Moreover, a linker should present the antigen properly to the immune system. Exemplary linkers include, but are not limited to, thioether linkages and polyamino acids like polyglycine having about 2 to about 20, preferably about 4 to about 12 amino acids, preferably glycine. Alternatively, short peptides having multiple amino acid sequences, such as GFLG, also can be used. In another example, the linkage can be a polyethylene glycol polymer having about 2 to 15, preferably 4 to 8 ethylene glycol moieties.

In some embodiments, linkers associate the antigen to an oligotuftsin derivative via one or both of the lysine (K) residues of the scaffold.

The bioconjugates of the present invention can be preferably used in a pharmaceutical composition which comprises the bioconjugates. In a preferred embodiment the pharmaceutical composition is a vaccine wherein the bioconjugate is in a stabilized solution which is pharmaceutically active. It is the intention of the vaccine to raise antibodies against the epitope contained within the bioconjugate. In one embodiment, an immunogenic composition is provided that comprises the disclosed pharmaceutical composition and an adjuvant.

In a further preferred embodiment of the present invention, the pharmaceutical compositions comprise at least one adjuvant which boosts the immune response. As noted below, a variety of suitable adjuvants are known in the art.

Conventional pharmaceutically acceptable carriers, excipients, buffers or diluents, may be included in vaccine compositions of this invention. Generally, a vaccine composition in accordance with the present invention will comprise an immunologically effective amount of the bioconjugate, and optionally an adjuvant, in conjunction with one or more conventional pharmaceutically acceptable carriers and/or diluents. In this regard, an "immunologically effective amount" denotes an amount sufficient to induce antibodies. An extensive, though not exhaustive list of adjuvants can be found in Cox and Coulter, "Advances in Adjuvant Technology and Application", in Animal Parasite Control Utilizing Biotechnology, Chapter 4, Ed. Young, W.K., CRC Press 1992, and in Cox and Coulter, "Adjuvants - A Classification and Review of Their Modes of Action", Vaccine 15(3), 248- 256, 1997. As used herein "pharmaceutically acceptable carriers and/or diluents" include any and all solvents, dispersion media, aqueous solutions, coatings, antibacterial and antifungal agents, isotonic and absorption delaying agents and the like. The use of such media and agents for pharmaceutical active substances is well known in the art and is described by way of example in Remington's Pharmaceutical Sciences, 18th Edition, Mack Publishing Company, Pennsylvania, U.S.A.

In another embodiment, a diagnostic test is provided for in vitro diagnosis of Alzheimer disease. The diagnostic test comprises a bioconjugate which serves as an antigen for the detection of specific antibodies directed against an epitope containing an oligomeric assembly of peptides having the amino acid sequences of Aβ (30-37) [SEQ ID NO. 5], Aβ (29-37) [SEQ ID NO. 6]; Aβ (28-37) [SEQ ID NO. 7]; Aβ (27-37) [SEQ ID NO. 8]; Aβ (26-37) [SEQ ID NO. 9]; Aβ (25-37) [SEQ ID NO. 10]; Aβ (24-37) (SEQ ID NO. 11]; Aβ (23-37) [SEQ ID NO. 12], Aβ (22-37) [SEQ ID NO. 13]; Aβ (21-37) [SEQ ID NO 4]; Aβ (20-37) [SEQ ID NO 14]; Aβ (19-37) [SEQ ID NO 15]; Aβ (18-37) [SEQ ID NO 16]; Aβ (17-37) [SEQ ID NO 17]; Aβ (16-37) [SEQ ID NO 18]; Aβ (15-37) [SEQ ID NO 19]; Aβ (14-37) [SEQ ID NO 20]; Aβ (13-37) [SEQ ID NO 21]; Aβ (12-37) [SEQ ID NO 22]; or Aβ (11-37) [SEQ ID NO 23]. Such oligomeric assemblies can comprise homo- or hetero-oligomers of said peptides. Suitable diagnostic tests are immunological tests like ELISA tests, Western Blots or Line Assays.

In another embodiment, methods are provided for the *in vitro* diagnosis of Alzheimer's disease in a patient comprising contacting a sample obtained from said patient with a bioconjugate of the present invention and detecting the binding of specific antibodies to said bioconjugate. The binding of antibodies to the bioconjugate can be shown by using antibodies directed against human antibodies. When IgG antibodies are detected the anti-antibody may be an antibody directed against the Fc fragment of human IgG antibodies. Similarly IgM antibodies may be detected by use of an anti-antibody directed against the Fc fragment of IgM. Such antibodies are commercially available and are usually produced in suitable animals like goats, sheep or horses.

Furthermore, the present invention relates to the use of the bioconjugates as described here in more detail for the vaccination of patients suffering from Alzheimer disease. Usually the bioconjugates can be administered to persons suffering from Alzheimer disease or patients who are prone to suffer from Alzheimer disease at least once, preferably, however, said bioconjugates are administered several times, preferably 2 to 6 times within a well-defined period of time. A suitable vaccination scheme is to apply the bioconjugates of the present invention three times whereby the second application is performed about three weeks after the first administration and the third administration is performed about six weeks after the first administration.

The present invention is described in more detail by referring to the figures.

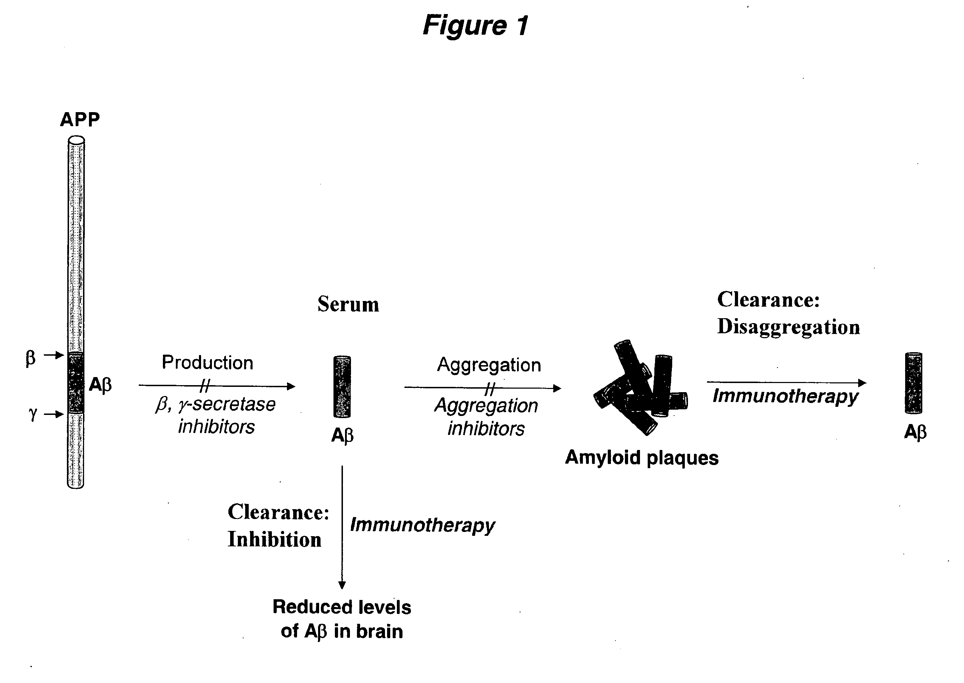

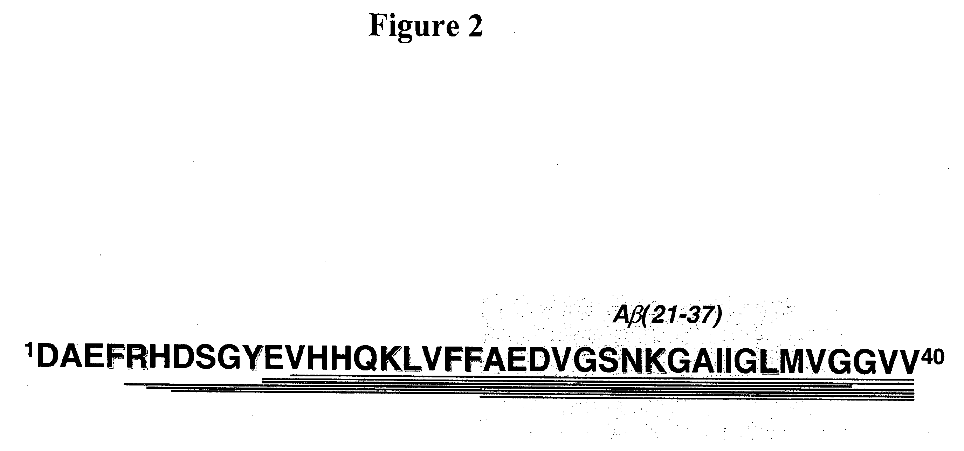

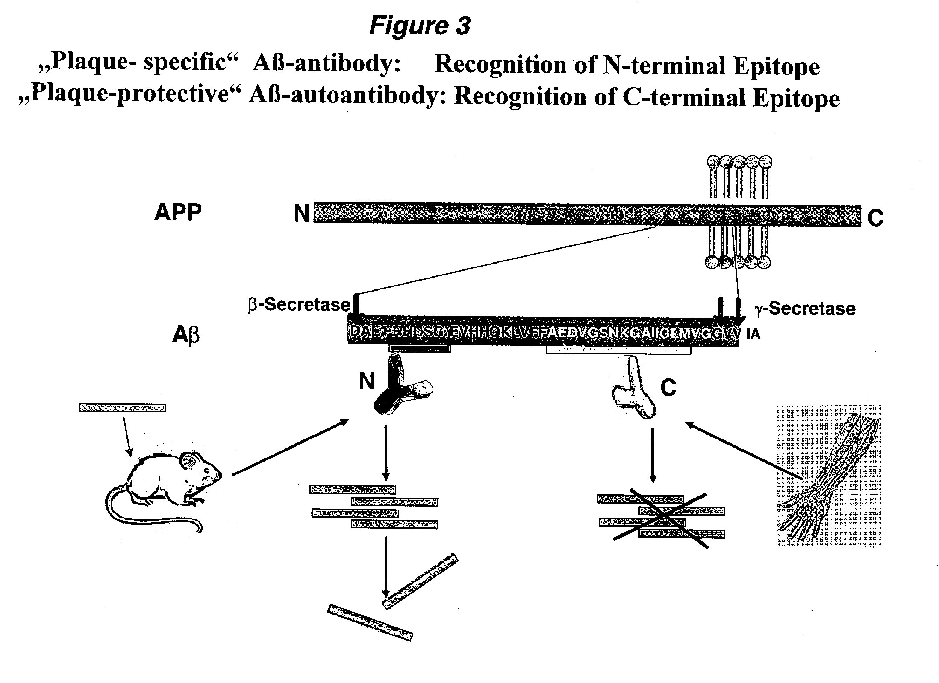

The immunotherapeutic principles based on Aβ-plaque-disaggregating and -inhibiting antibodies, and the identification and characterisation of the "plaque-specific" and "plaque-protecting" Aβ-epitopes are summarized in **Figures 1** - **3****.**

For the determination of the Aβ-epitopes, both epitope extraction and -excision mass spectrometry techniques were employed, using a combination of matrix-assisted laser desorption/ionization- (MALDI) and electrospray ionization- (ESI) MS and high resolution FTICR-MS; these methods enable determination of polypeptides with mass ranges up to several kDa and mass accuracies at the low ppm level, and were used for the structural characterization of Aβ-epitope bioconjugates.

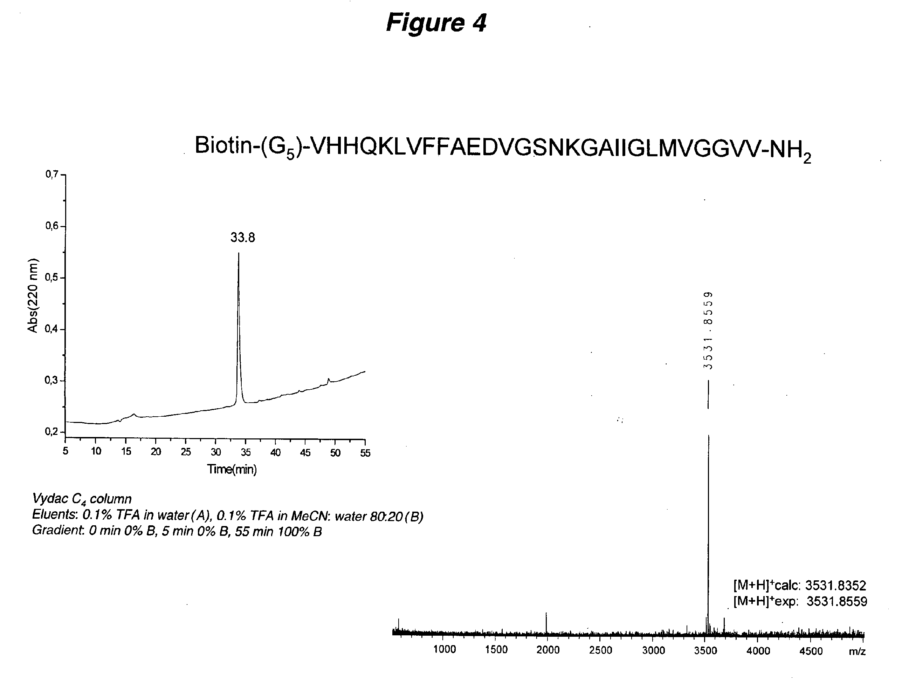

Based on the exact knowledge of the Aβ-epitope sequences, biotinylated penta-glycyl-Aβ(12-40) and Cys-pentaglycyl-Aβ(12-40) peptides were synthesized, comprising the Aβ-autoantibody epitope (but excluding binding plaque-specific antibodies), and used for (i), Aβ-autoantibody determinations, and (ii) affinity-isolation of antibodies (**Figures 4 - 6**). Since Aβ(12-40) contains two internal lysine residues, an improved affinity- purification protocol was used with incorporating a cysteine residue attached to the N-terminus, thus ensuring that the peptides will be oriented on the column support in an identical manner after immobilisation. The azlactone-activated support contains an iodoacetyl group (UltraLink; Perbio) at the end of a 15-carbon alkyl spacer arm, to provide a stable thioether linkage and reduce steric strain in antibody binding.

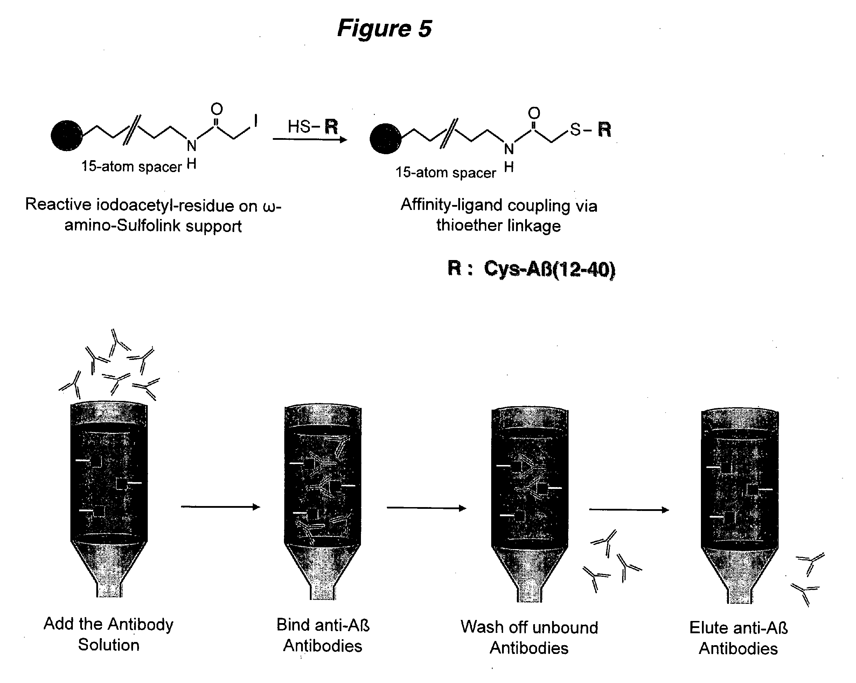

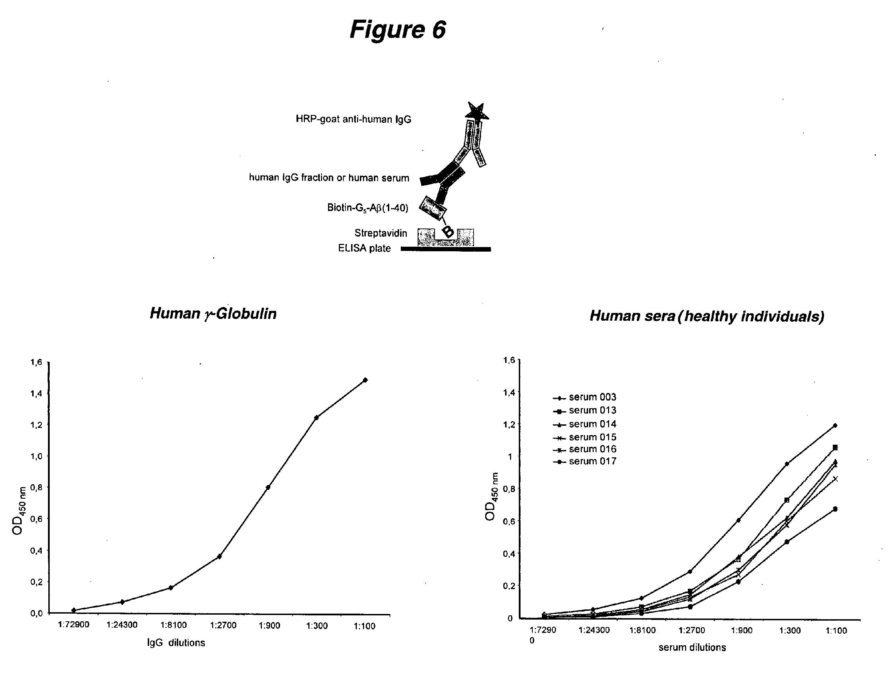

Using the Cys-Aβ-(12-40) affinity matrix, epitope-specific Aβ-autoantibody isolation and purification was performed as outlined in **Figure 5****.** A 0.5 ml aliquot of the Cys-Aβ(12-40)-coupled support was packed into a column (2.5 ml, MoBiTec, Göttingen, Germany) and equilibrated with 20 ml PBS (0.1 M sodium-phosphate, 0.15 M NaCl, pH 7.2) . The support was transferred into a 15 ml Falcon vial using 5 ml PBS and mixed with 5 ml IVIgG. The diluted gel slurry was slowly rotated overnight at 4 °C, and the suspension then transferred to a column using the effluent to completely rinse the matrix back into the column. The column was washed eight times with 10 ml PBS followed by 2 wash cycles with 10 ml ultrapure water. The affinity-bound antibodies were eluted from the column with 10 x 0.5 ml 0.1 % TFA. IgG preparation for further experiments was performed using two different protocols:
(a) the first procedure involved adjusting the solution to pH 7 for each fraction collected using 0.5 M NaH₂PO₄, pH 8.8, in order to maintain structural integrity of the "native" antibodies for affinity determinations (**Figure 6**);
(b) for 2D-PAGE separations of heavy and light chains the elution of the bound IgG was not accompanied by pH adjustment, in order to reduce the salt content present in the sample subjected to isoelectric focusing. Antibody concentrations in the elution fractions were determined by the micro-BCA method (Pierce; Perbio, Bonn, Germany). A stock solution of 2 mg/ml of bovine albumin supplied within the Micro BCA^{™} Kit was used to prepare fresh standard dilutions within the range 40-0.5 µg/ml. The antibodies eluted between fractions 1 to 6, with highest concentrations in fractions 1 and 3. For quantification of each set of 10 elution fractions, fresh albumin standard dilutions were prepared, and results were obtained at 562 nm with a VICTORII ELISA reader. A positive control for the performance of the antibody column was performed as described in **Figure 5****.**

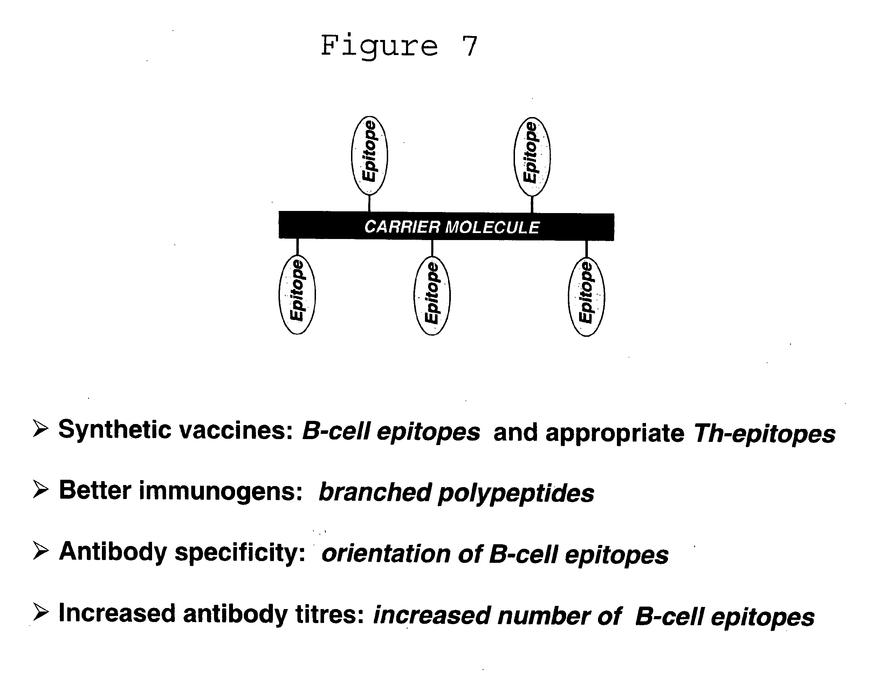

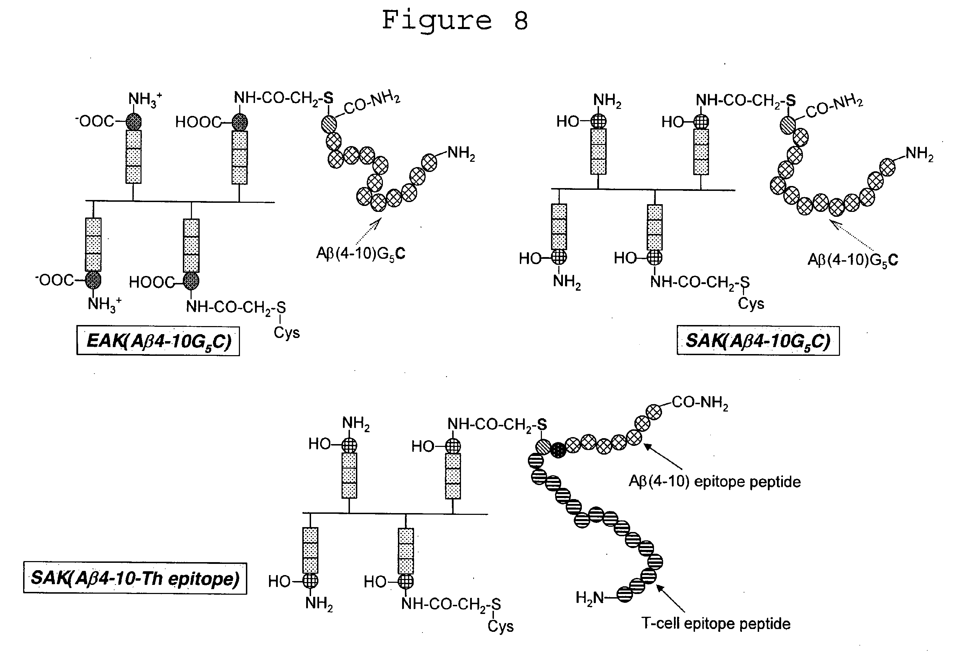

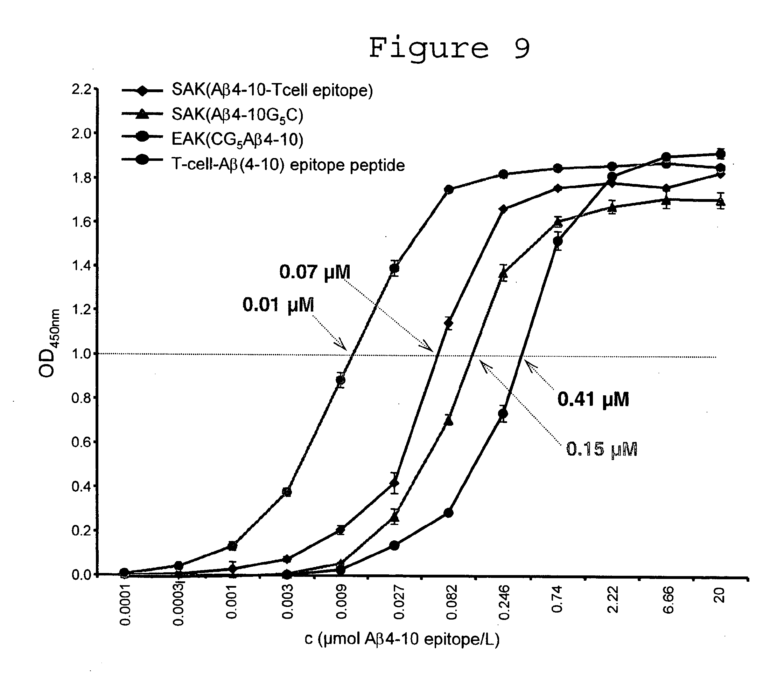

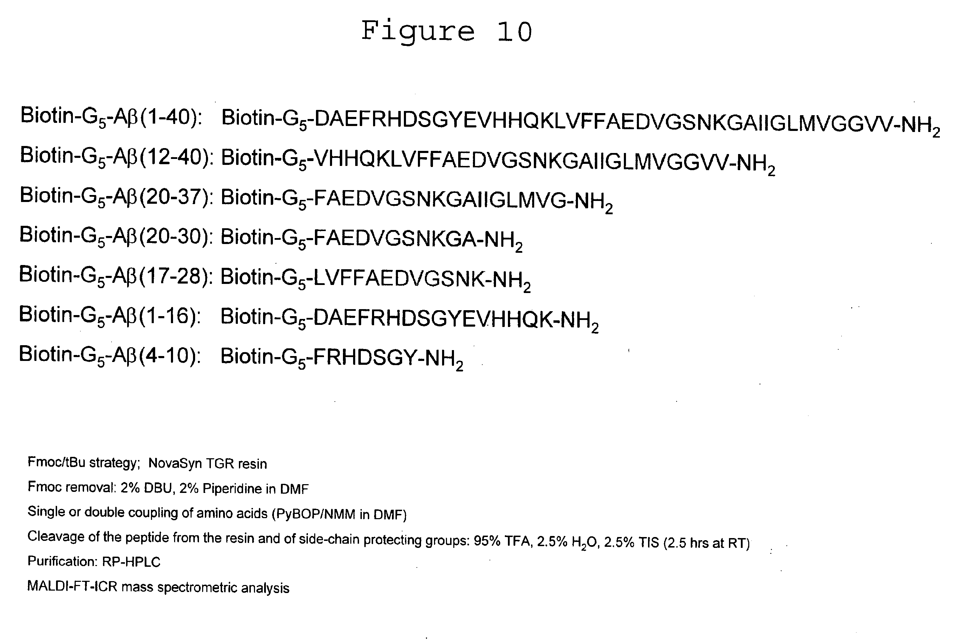

A general scheme for the design of synthetic peptide based vaccine conjugates by incorporation of multiple copies of a B-cell epitope and a promiscuous helper T-cell epitope in a polymer-carrier architecture is shown in **Figure 7****.** In a first set of carriers, bioconjugates were prepared in which Aβ(4-10) and combined T-helper cell-Aβ(4-10) epitope peptides were attached through a thioether bond to branched chain polymeric polypeptides, SAK and EAK. These polymeric carriers are based on a poly[L-Lys] backbone and contain short side chains composed of 3 DL-Ala residues, and Ser or Glu residues at the end of the branches (**Figure 8**). In these conjugates the epitope peptide, elongated by a Cys-Gly₅ sequence at the N-terminus or Gly₅Cys- at the C-terminus was attached to the chloroacetylated carrier *via* a thioether ligation. A third construct contained polymeric carrier (EAK) and an epitope chimera was also prepared in which a promiscuous T-helper cell epitope and the Aβ- epitope were joined by a dipeptide (Ahx-Cys) spacer (FFLLTRILTIPQSLD-AhxC-Aβ- epitope). Binding studies by ELISA using a mouse anti-Aβ(1-17) monoclonal antibody showed that conjugates comprising the Aβ(4-10) epitope peptide and a T-helper cell-Aβ(4-10) peptide construct had high affinities with K_{D} values in the nanomolar range (**Figure 9**).

A series of C-terminal Aβ-peptides (epitope peptide), elongated by a Cys-Gly₅ at the N-terminus or Gly₅Cys- at the C-terminal was attached to the chloroacetylated carrier *via* a thioether bond. A third construct contained polymeric carrier (EAK) and an epitope chimera in which a promiscuous T-helper cell epitope and the Aβ(4-10) epitope were joined by a dipeptide (Ahx-Cys) spacer (FFLLTRILTIPQSLD-AhxC-FRHDSGY).

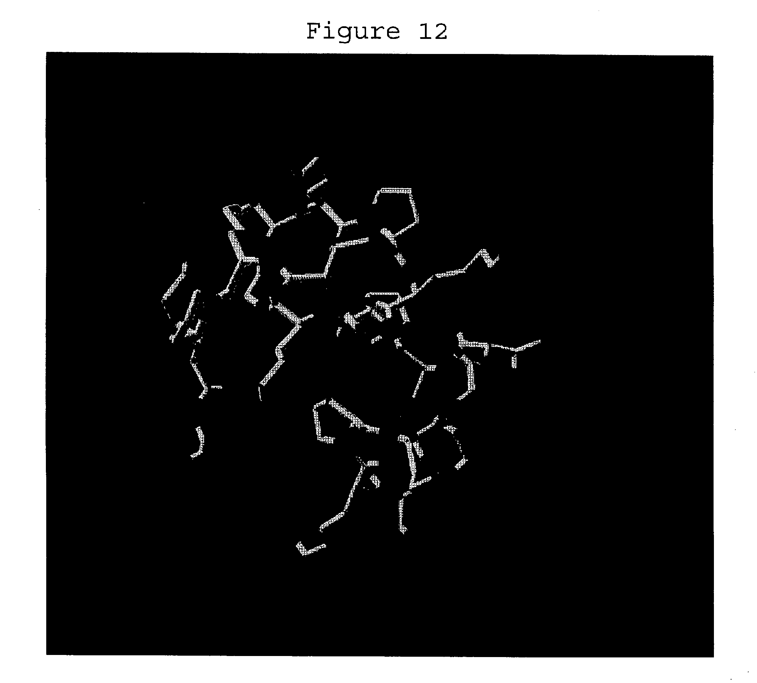

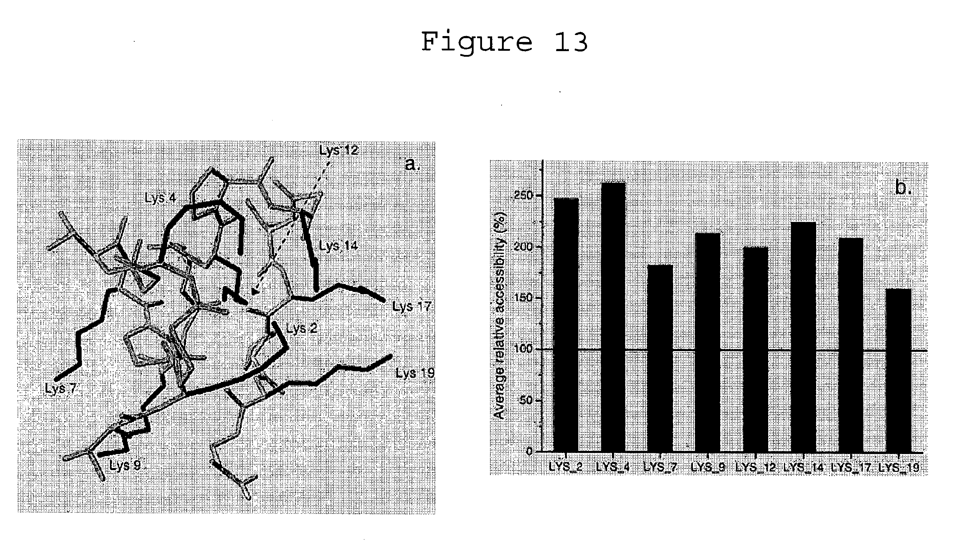

In the present invention a novel group of sequential oligopeptides based on tuftsin for conjugation to Aβ-antibody epitopes has been developed. Tuftsin is a sequential oligopeptide consisting of four tandem repeat units of a pentapeptide (Ac-[TKPKG]₄-NH₂, Ac-T20) derived from the canine tuftsin. Oligotuftsins such as the tetratuftsin derivative T20 (**Figures 11-14**) are non-toxic, non-immunogenic and exhibit tuftsin like biological properties, e.g. immunostimulatory activity and chemotactic activity. Oligotuftsin derivatives consisting of tandem pentapeptide repeat units [TKPKG]ₙ (n = 2, 4, 6 etc.) derived from the canine tuftsin sequence TKPK extended by a C-terminal Gly. The tuftsin derivative employed here has been derived from the canine tuftsin sequence (TKPK), which differs from human tuftsin only in the C-terminal amino acid, where arginine has been replaced by lysine. This mutation has no adverse effect on the biological activity; however, the additional lysine residue in the sequence offers a suitable set of functional groups for the attachment of Aβ-epitopes (as well as other biologically active compounds), by providing homogeneous equal surface accessibilities of lysine-amino groups in the oligotuftsin structure (**see** **Figures 12****,** **13**). Another advantage of this carrier is its hydrophilic character which provides high water solubility of bioconjugates.

Chemoselective ligation using thioether linkages was used for the attachment of cysteine modified epitope peptides to the chloroacetylated oligotuftsin derivatives, Ac-[TKPK(ClAc)G]₄-NH₂ and its version containing a pentaglycine spacer, Ac-[TKPK(ClAc-GGGGG)G]₄-NH₂. We have previously performed successfully synthesis of several Aβ(4-10)-conjugates in which the epitope (with or without Ala or β-Ala flanking residues) was attached to the tetratuftsin carrier (or its version elongated by a helper T-cell epitope (Ac-FFLLTRILTIPQSLD-[TKPK(H-X₂FRHDSGYX₂)G]₄-NH₂, where X= a chemical bond, Ala or β-Ala) via amide bonds).

The present invention relates in a preferred embodiment to the development of bioconjugates comprising single and multiple (oligomeric) copies of the Aβ(21-37) autoantibody epitope by its attachment to oligopeptide carriers. In the following synthesis, structural and immuno-analytical characterizations of bioconjugates containing multiple copies of Aβ(21-37) epitope are described. Furthermore, conjugates containing shorter Aβ-sequences (N-terminal or C-terminal truncated Aβ-peptides) were prepared and investigated, in order to elucidate the molecular requirements and mechanism, and the specificity of the binding of the Aβ(21-37) autoantibody epitope.

For the synthesis of the chloroacetylated oligotuftsin carriers, three types of derivatives containing different oligopeptide spacers (e.g., pentaglycine) were prepared and explored for reactivity and solubility of the bioconjugates obtained:

| | |
|---|---|
| Ac-T20(ClAc)₄: | Ac-[TKPK(ClAc)G]₄-NH₂ |
| Ac-T20(ClAc-GFLG)₄: | Ac-[TKPK(ClAc-GFLG)G]₄-NH₂ |
| Ac-T20(ClAc-G₅)₄: | Ac-[TKPK(ClAc-GGGGG)G]₄-NH₂ |

Chloroacetylated (ClAc-) oligotuftsin derivative carriers were synthesized manually, by SPPS according to the Boc/Bzl strategy using MBHA resin (1.2 mmol/g capacity) as a support, and by using the following Boc-protected amino acid derivatives: Boc-Gly-OH, Boc-Lys[Z(2Cl)]-OH, Boc-Lys(Fmoc)-OH, Boc-Pro-OH, Boc-Thr(Bzl)-OH, Boc- Leu-OH·H₂O and Boc-Phe-OH. The general synthesis protocol was as follows: (i) deprotection with 33% TFA/DCM (2 + 20 min); (ii) DCM washing (5 × 0.5 min); (iii) neutralization with 10% DIEA/DCM (4 × 1 min); (iv) DCM washing (4 × 0.5 min); (v) coupling of 3 equiv of Boc-amino acid derivative - DCC - HOBt in DCM-DMF 4 : 1 or 1 : 4 (v/v) mixture, depending on the solubility of Boc-amino acid derivatives (60 min); (vi) DMF washing (2 × 0.5 min); (vii) DCM washing (2 × 0.5 min); (viii) ninhydrin or isatin assay. The acetylation of the N-terminal end was carried out with acetic anhydride (1 ml) and DIEA (1 ml) in DMF (3 ml) for 30 min. The N^{ε}-amino group of Lys residues was chloroacetylated using 5 equiv/Lys of chloroacetic acid pentachlorophenyl ester in DMF-DCM (1 : 1, v/v) after removal of the N^{ε}-Fmoc groups with a 2% DBU and 2% piperidine mixture in DMF (2 + 2 + 5 + 10 min). The coupling reaction was carried out for 2-3 h. In case of the tetratuftsin derivative containing a GFLG or GGGGG spacer, the spacer was built up on Lys side-chains first by using 12 equiv of Boc-amino acid derivative for the four Lys residues, and then the peptide was chloroacetylated. The chloroacetylated peptides were cleaved from the resin by liquid HF in the presence of m-cresol and p-thiocresol (HF-m-cresol-p-thiocresol = 10 ml: 0.5 ml: 0.5 g) for 1.5 h at 0°C. The crude products were purified by HPLC and analyzed by mass spectrometry.

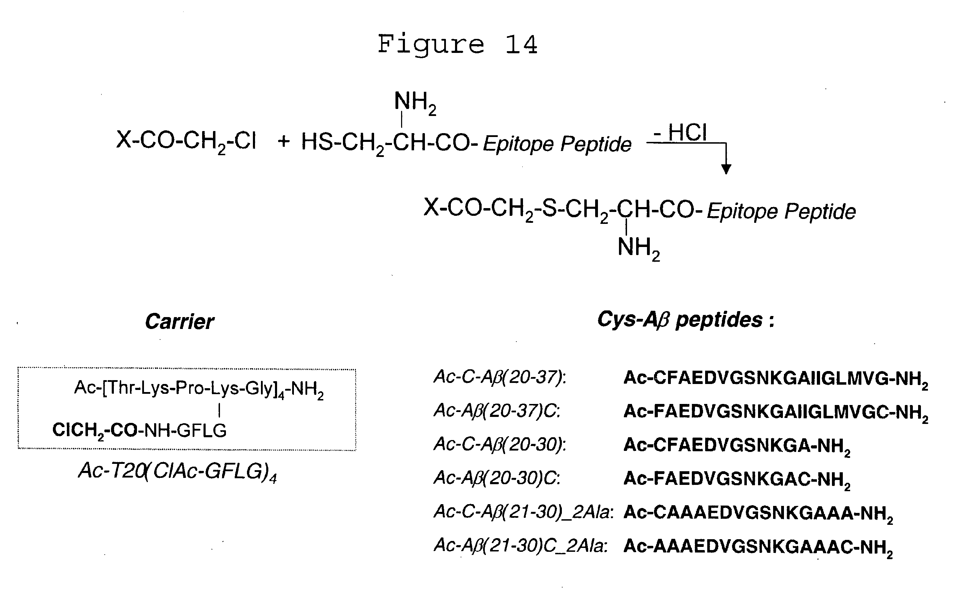

A series of cysteine- containing Aβ-epitope peptides was prepared for conjugation to the tetratuftsin carriers, according to the general scheme shown in **Figure 14****:**

| | |
|---|---|
| Ac-C-Aβ(20-30) | Ac-CFAEDVGSNKGA-NH₂ |
| Ac-Aβ(20-30)-C | Ac-FAEDVGSNKGAC-NH₂ |
| Ac-C-Aβ(21-30)_2Ala flank: | Ac-CAAAEDVGSNKGAAA-NH₂ |
| Ac-Aβ(21-30)-C_2Ala flank: | Ac-AAAEDVGSNKGAAAC-NH₂ |
| Ac-C-Aβ(20-37) | Ac-CFAEDVGSNKGAIIGLMVG-NH₂ |
| Ac-Aβ(20-37)-C | Ac-FAEDVGSNKGAIIGLMVGC-NH₂ |
| Ac-C-Aβ(21-37) | Ac-CAEDVGSNKGAIIGLMVG-NH₂ |
| Ac-C-TEG-Aβ(21-37) | Ac-C-TEG-AEDVGSNKGAIIGLMVG-NH₂ |
| (TEG = tetraethyleneglycol) | |

In a general synthesis procedure, cysteine- containing Aβ peptides were prepared on a NovaSyn TGR resin (0.23 mmol/g) using Fmoc/*t*Bu chemistry, using a semi-automated Peptide Synthesizer EPS-221 (INTAVIS, Germany). The following side-chain protected amino acids were employed: Fmoc-Lys(Boc)-OH, Fmoc-Asn(Trt)-OH, Fmoc-Ser(tBu)-OH, Fmoc-Asp(OtBu)-OH, Fmoc-Glu(OtBu)-OH, and Fmoc-Cys(Trt)-OH. The protocol was as follows: (i) DMF washing (3 × 1 min), (ii) deprotection with 2% DBU, 2% piperidine in DMF (15 min), (iii) DMF washing (6 × 1 min), (iv) coupling of 5 equiv of Fmoc amino acid : PyBOP : NMM in DMF (45 min), (v) DMF washing (3 × 1 min). After completion of the synthesis, the N-terminal end was acetylated with acetic anhydride (1 ml) and DIEA (1 ml) in DMF (3 ml) for 30 min. The peptides were cleaved from the resin using TFA, triethylsilane and deionized water (95 : 2.5 : 2.5, v/v/v) for 2.5 h at room temperature. Due to their high hydrophobicity, the epitope peptides Ac-C-Aβ(20-37), Ac-Aβ(20-37)-C, Ac-C-Aβ(21-37), Ac-C-TEG-Aβ(21-37) were synthesized using a double coupling procedure for each amino acid. Ac-C-Aβ(21-37), Ac-C-TEG-Aβ(21-37) peptides were also synthesized manually on a Rink-Amide MBHA resin (0.72 mmol/g coupling capacity). All peptides were purified by HPLC and analyzed by MALDI-MS.

Conjugations of Cys-containing epitope peptides to the oligotuftsin carriers were obtained by first dissolving the chloroacetylated oligopeptide carriers (Ac-[TKPK(ClAc)G]₄-NH₂, Ac-[TKPK(ClAc-GFLG)G]₄-NH₂) in 0.1 M Tris buffer (pH 8) at 1 mg/ml. Cysteine containing Aβ(20-30) and Aβ(20-37) peptides in solid form were then added to the carrier solutions in small portions (2-3 mg every 10-15 min). Eight equivalents of Cys-peptide (2-fold excess corresponding to the CIAc-groups) were used in each case. The conjugation reactions were carried out for 24 hrs at room temperature in a tightly closed tube, and the reaction was monitored by analytical RP-HPLC and mass spectrometry. After completion, the reaction mixture was acidified with TFA to pH 3, and bioconjugates were separated by semipreparative HPLC. For the Aβ(21-37)-conjugate, approximately 5 mg of Ac-C-Aβ(21-37) peptide was dissolved in 6-8 mL of a 1 mg/mL solution of sodium carbonate : acetonitrile = 1:1 (v/v), pH 11, and then 1 mg of chloroacetylated oligotuftsin derivative carrier Ac-T20(ClAc-GFLG)₄ was added (the peptide was used in a 2-3-fold excess corresponding to the ClAc-groups). After 24 hrs, the reaction mixture was acidified with TFA to pH 3 and purified by semipreparative RP-HPLC.

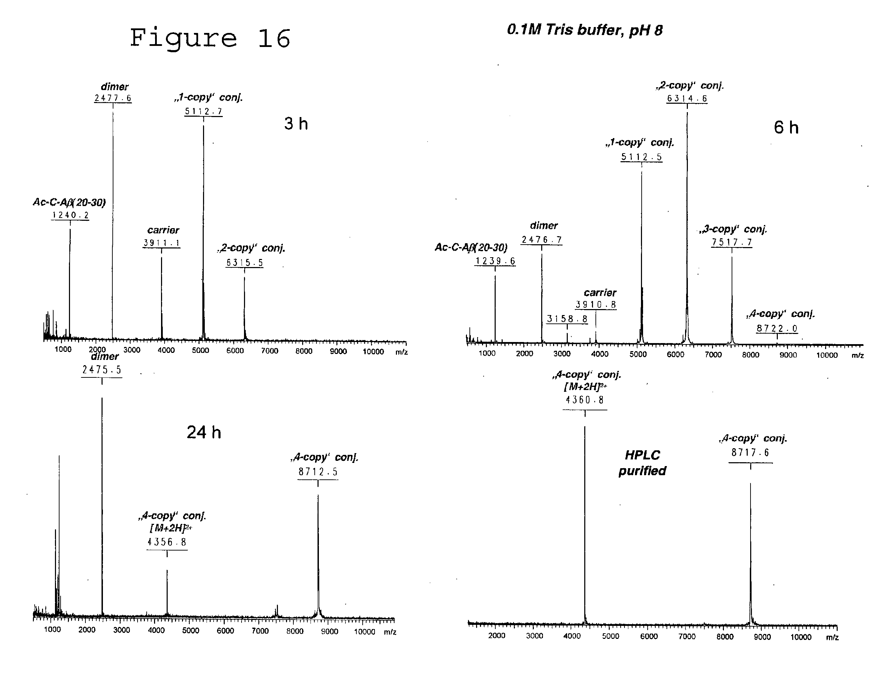

After lyophilization, the HPLC-purified bioconjugates were analyzed by MALDI mass spectrometry, as illustrated in **Figures 15** **and** **16****.** The MALDI-MS data, as well as molecular weight determinations by ESI-MS (not shown) confirm the structures of the tetratuftsin-Aβ(21-37) conjugate to comprise a mixture of the tri- and tetra-copy oligomers (mol. weights. 11,475, and 9,548); identical tri/tetrameric conjugates were obtained for the Aβ(20-30) conjugate. Further separations of the trimeric and tetrameric conjugates could not be obtained with the present HPLC conditions, and are currently underway with new separation matrices.

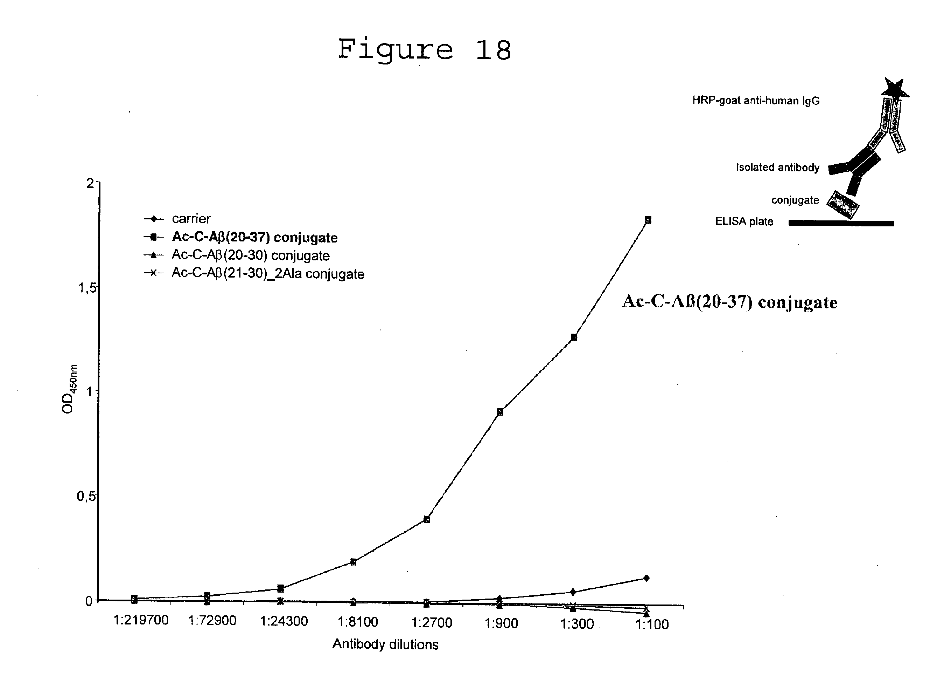

Binding affinity studies to (i) human IgG and (ii), the affinity-isolated Aβ-autoantibody, using indirect ELISA are compared in **Figures 17** **and** **18****.** In both cases, high and specific affinities were only obtained for the oligotuftsin tri-/tetrameric Aβ(21-37) conjugate. In contrast, no (or only very weak) binding affinity was obtained for the linear ("monomeric") Aβ(21-37) epitope peptide in indirect ELISA, furthermore the linear peptide showed only very poor solubility, under conditions employed. The Aβ(20-30) peptide did not show any binding affinity, neither the tri/tetra-oligotuftsin -conjugates (**Figure 18**), nor the linear peptide. Likewise, the peptides Aβ(25-35) and Aβ(31-40) did not show any binding to the Aβ-autoantibodies. These results are in full agreement with, and show the recognition of the Aβ-autoantibodies
(i) by low molecular weight oligomers of the carboxy-terminal Aβ-epitope;
(ii) the epitope specificity of the Aβ(21-37) sequence; while the corresponding partial sequences such as Aβ(20-30) are lacking any of binding affinity.

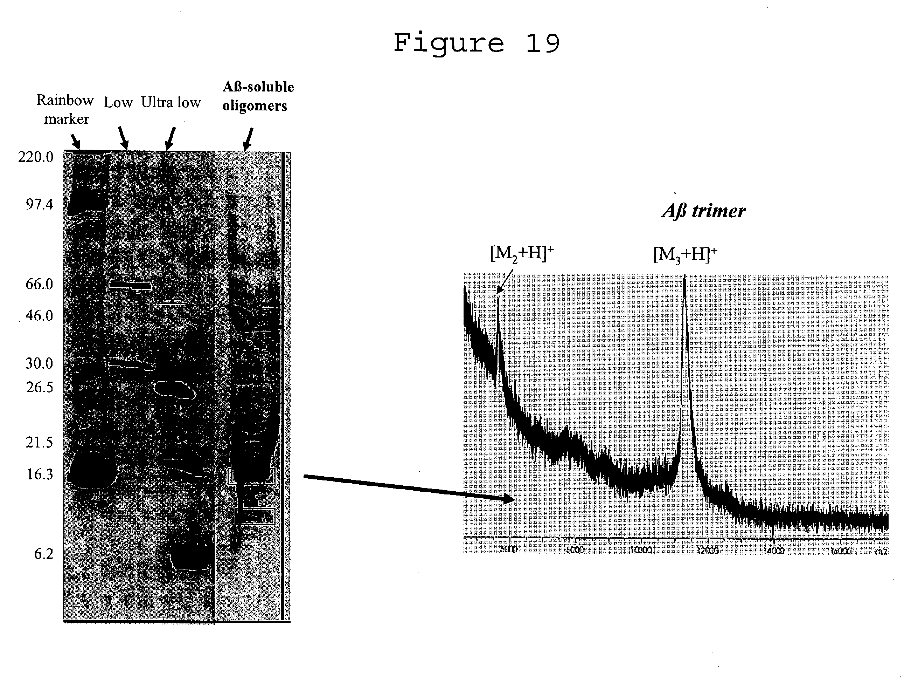

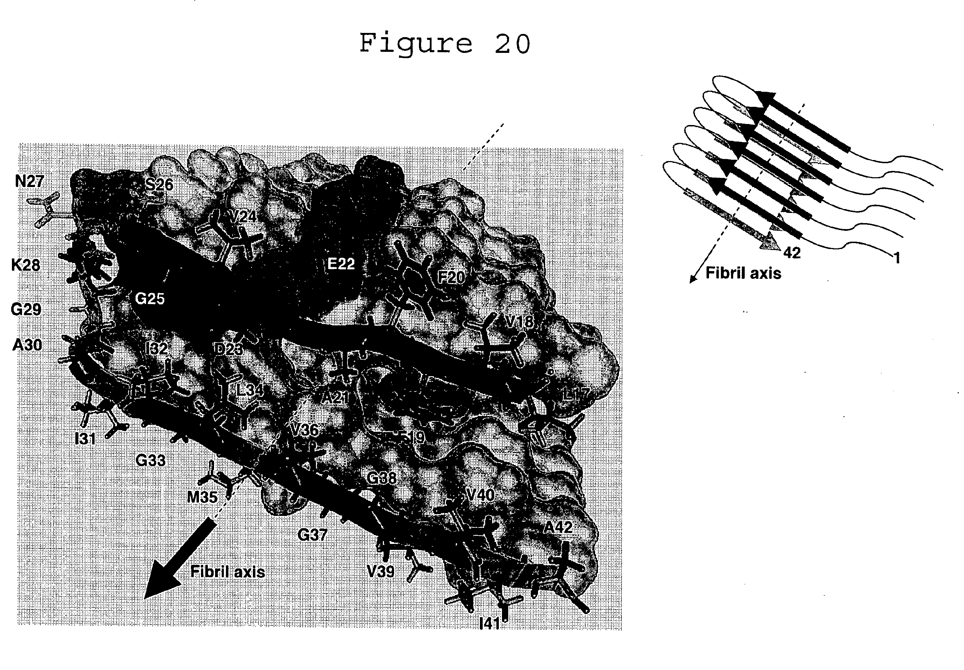

The oligomer- specificity of the autoantibodies is further supported by the direct mass spectrometric identification (MALD-MS) of a trimeric Aβ-oligomer which could be isolated after immunoprecipitation from the Aβ-autoantibody, and separated by gel electrophoresis (**Figure 19**). Furthermore, these results are in full agreement with the recently published structure model of Aβ-fibrils (**Figure 20**), derived from a combination of NMR and molecular modeling studies, which show that large Aβ-fibrils comprise two parallel β-strands of Aβ(18-26) and Aβ(31-40), that are separated by a turn within the Aβ(27-30) residues.

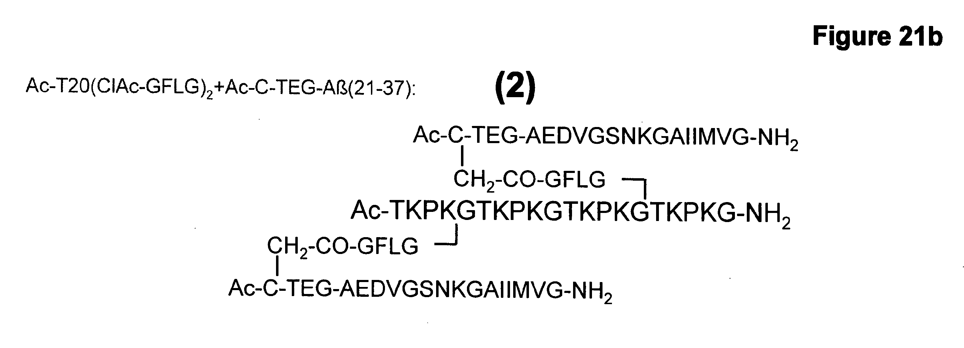

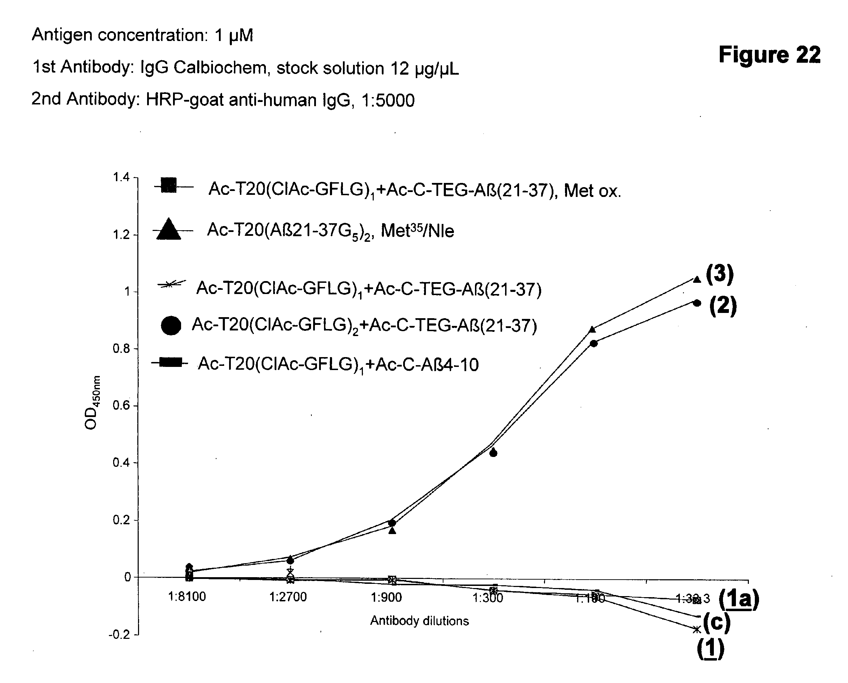

The recognition specificity of the Aβ-autoantibodies was tested further and ascertained by synthesis and affinity determinations of oligotuftsin conjugates in which a defined number of Aβ-epitope peptides were incorporated. Corresponding bioconjugates, containing either 1 or 2 copies of the Aβ(21-37), but otherwise having identical structures are shown in **Figure 21****(a-c).** Linkages between Aβ-epitope and the peptide carrier were made with different types of spacer groups, (i) short oligopeptide -GFLG- spacer, (ii) oligoethylene glycol, and (iii) pentaglycine, in order to ensure sufficient flexibility of binding topology for the Aβ-epitopes. In **Figure 22****,** binding affinities to human IgG are compared for scaffolds comprising one antigen and scaffolds comprising 2 antigens. As shown in the figure, only scaffolds with at least 2 copies of the Aβ-peptide produced measurable binding.

Thus, bioconjugates of Aβ are provided that
(i) specifically bind to Aβ-neuroprotective autoantibodies with high affinity;
(ii) constitute specific and high-affinity scaffolds for the detection and isolation of Aβ-oligomer-specific antibodies;
(iii) enable new specific diagnostic determinations of Aβ-antibodies; and
(iv) constitute lead structures for the design and preparation of new Aβ-oligomer specific immunogens and vaccine conjugates for immunotherapy of AD and AD-related diseases.

The invention provides the direct identification, and access to molecular chemical structures, of neuroprotective and therapeutic antibodies recognising soluble, oligomeric molecular forms of Aβ. Furthermore, the new bioconjugates provide specific and sensitive tools for probing the mechanism of action of Aβ-antibodies, and are expected to contribute to both the biochemical understanding and biomedical applications of Aβ-antibodies.

A wide range of biochemical, analytical and biomedical applications are envisaged, including:
(i) the use of Aβ bioconjugates for the study of Aβ-aggregation and understanding of amyloidogenic protein-peptide interactions;
(ii) the application to affinity-isolation of specific Aβ-antibodies and their interaction partners from biological and cellular material; and
(iii) the design and development of new specific aggregation inhibitors for AD therapy.

### Examples

### Example 1: Synthesis of oligotuftsin-Aβ-(21-37) oligomer-conjugates

The structure of the branched conjugates is shown in Figure 23a-e.

### a) Synthesis of oligotuftsin derivative, Ac-OT20

Oligotuftsin derivative **Ac-[TKPKG]₄-NH₂ (Ac-OT20)** was synthesized manually by SPPS according to the Boc/Bzl strategy using MBHA resin (1.04 mmol/g capacity) as a support. The following Boc-protected amino acid derivatives were used: Boc-Gly-OH, Boc-Lys(CIZ)-OH, Boc-Pro-OH, Boc-Thr(Bzl)-OH. The following synthetic protocol was employed: (i) deprotection with 33% TFA/DCM (2 + 20 min); (ii) DCM washing (5 × 0.5 min); (iii) neutralization with 10% D[EA/DCM (4 × 1 min); (iv) DCM washing (4 × 0.5 min); (v) coupling of 3 equivalents of Boc-amino acid derivative using DCC/HOBt in DCM-DMF 4 : 1 (60 min); (vi) DMF washing (2 × 0.5 min); (vii) DCM washing (2 × 0.5 min); (viii) ninhydrin or isatin assay to assess the presence of free amines. Acetylation of the N-terminus was carried out for 1 h with acetic anhydride (1 mL) and DIEA (1 mL) in DMF (3 mL).

### b) Synthesis of chloroacetylated oligotuftsin derivative carriers

| | |
|---|---|
| Ac-OT20(CIAc)₄: | **Ac-[TKPK(ClAc)G]₄-NH₂** |
| Ac-OT20(CIAc-GFLG)₄: | **Ac-[TKPK(ClAc-GFLG)G]₄-NH₂** |
| Ac-OT20(CIAc-G₅)₄: | **Ac-[TKPK(ClAc-GGGGG)G]₄-NH₂** |

Chloroacetylated (CIAc-) oligotuftsin derivative carriers were synthesized manually, by SPPS, according to the Boc/Bzl strategy using MBHA resin (1.2 mmol/g capacity) as a support. The following Boc-protected amino acid derivatives were used: Boc-Gly-OH, Boc-Lys(CIZ)-OH, Boc-Lys(Fmoc)-OH, Boc-Pro-OH, Boc-Thr(Bzl)-OH, Boc-Leu-OH•H₂O and Boc-Phe-OH. The protocol was as follows: (i) deprotection with 33% TFA/DCM (2 + 20 min); (ii) DCM washing (5 × 0.5 min); (iii) neutralization with 10% DIEA/DCM (4 × 1 min); (iv) DCM washing (4 × 0.5 min); (v) coupling of 3 equiv of Boc-amino acid derivative - DCC - HOBt in DCM-DMF 4 : 1 or 1 : 4 (v/v) mixture, depending on the solubility of Boc-amino acid derivatives (60 min); (vi) DMF washing (2 × 0.5 min); (vii) DCM washing (2 × 0.5 min); (viii) ninhydrin or isatin assay. The acetylation of the N-terminal end was carried out with acetic anhydride (1 mL) and DIEA (1 mL) in DMF (3 mL) for 30 min. The N^{ε}-amino group of Lys residues was chloroacetylated using 5 equiv/Lys of chloroacetic acid pentachlorophenyl ester in DMF-DCM (1 : 1, v/v) after removal of the N^{ε}-Fmoc groups with a 2% DBU and 2% piperidine mixture in DMF (2 + 2 + 5 + 10 min). The coupling reaction was carried out for 2-3 h. In case of the oligotuftsin derivative containing a GFLG or GGGGG spacer, the spacer was built up on Lys side-chains first by using 12 equiv of Boc-amino acid derivative for the four Lys residues, and then the peptide was chloroacetylated. The chloroacetylated peptides were cleaved from the resin by liquid HF in the presence of m-cresol and p-thiocresol (HF-m-cresol-p-thiocresol = 10 ml: 0.5 ml: 0.5 g) for 1.5 h at 0°C.

### c) Synthesis of chloroacetylated Ac-Tcell-OT20(ClAc)₄

Another chloroacetylated oligotuftsin derivative analogue, elongated at the N-terminus with a promiscuous helper T-cell epitope derived from a hepatitis B surface antigen (Ac-**FFLLTRILTIPQSLD-[TKPK(ClAc)G]₄-NH₂**), was synthesized on MBHA resin (0.67 mmol/g capacity). The following side chain protected N°-Boc-amino acid derivatives were used: Boc-Arg(Tos)-OH, Boc-Asp(OcHex)-OH, Boc-Lys(CIZ)-OH, Boc-Lys(Fmoc)-OH, Boc-Ser(Bzl)-OH, Boc-Thr(Bzl)-OH. The protocol of the synthesis was as follows: (i) DCM washing (3 x 0.5 min), (ii) Boc cleavage using 33% TFA in DCM (2+20 min), (iii) DCM washing (5 x 0.5 min), (iv) neutralization with 10% DIEA in DCM (4 x 1min), (v) DCM washing (4 x 0.5 min), (vi) coupling 3 equivalents of Boc-amino acid derivative-DCC-HOBt in DCM-DMF mixture (4:1 or 1:4 v/v depending on the solubility of the amino acid derivatives) (60 min), (vii) DMF washing (2 x 0.5 min), (viii) DCM washing (2 x 0.5 min), (ix) ninhydrine or isatin assay to assess the presence of free amines or imines. Due to the pronounced hydrophobic character of the helper T-cell epitope sequence (FFLLTRILTIPQSLD), double coupling of the amino acids was necessary for assembling the N-terminal part of this carrier. After completion of the synthesis, the acetylation of the N-terminus was carried out with acetic anhydride (1 mL) and DIEA (1 mL) in DMF (3 mL) for 1 hour at room temperature. Prior to the chloroacetylation of the appropriate Lys side chains with chloroacetic acid pentachlorophenyl ester (CIAcOPcp), the Fmoc protecting groups were removed with 2% DBU-2% piperidine in DMF (four times for 2+2+5+10 min). The peptide was cleaved from the resin simultaneously with the removal of the side-chain protecting groups using liquid HF (10 mL) in the presence of m-cresol (0.5 mL) and p-thiocresol (0.5 g) as scavengers.

### d) Synthesis of conjugate 5: Ac-Tcell-T20(Ac-C-Aβ20-30)₄

### Conjugation of Ac-C-Aβ(20-30) peptide to chloroacetylated oligotuftsin derivative carrier Ac-Tcell-T20(ClAc)₄

Chloroacetylated oligotuftsin derivative carrier Ac-FFLLTRILTIPQSLD-[TKPK(CIAc)G]₄-NH₂ (Ac-Tcell-T20(CIAc)₄) was dissolved in 0.1 M Tris buffer (pH =8.1) at a concentration of 2 mg/ml. Cysteine containing Aβ(20-30) peptide, in solid form, was added to the carrier solution in small portions (2-3 mg every 15-30 min). Twelve equivalents of Cys-peptide (3-fold excess corresponding to the CIAc-groups) were used. The conjugation was carried out for 36 hrs at RT in a tightly closed tube. After completion, the reaction mixture was acidified with TFA to pH 3 and the "4-copy" bioconjugate was separated by preparative RP-HPLC on a C₄ column.

### Synthesis of conjugate 10: Ac-Tcell-T20(Ac-C-TEG-Aβ21-37)_{3/4}

### Conjugation of Ac-C-TEG-Aβ(21-37) peptide to chloroacetylated oligotuftsin derivative carrier Ac-Tcell- T20(ClAc)₄

Chloroacetylated oligotuftsin derivative carrier Ac-FFLLTRILTIPQSLD-[TKPK(CIAc)G]₄-NH₂ (Ac-Tcell-T20(ClAc)₄) was dissolved in a mixture of 2 mg/mL sodium carbonate : acetonitrile = 1:1 (v/v) at a concentration of 1 mg/mL and then the Ac-C-TEG-Aβ21-37 peptide in solid form was added in only one portion to the carrier solution (3-fold excess corresponding to the CIAc-groups). The conjugation was carried out for 16 hrs at RT. The reaction mixture was first lyophilized and then the lyophilized powder was dissolved in a mixture of 100 µL DMSO, 200 µL eluent B (acetonitrile : water 80:20 (v/v) containing 0.1 % TFA) and 700 µL eluent A (0.1% TFA in water) and purified by preparative RP-HPLC on a C₄ column.

### Synthesis of conjugate 12: Ac-T20-GGGGG(Ac-C-TEG-Aβ21-37)₂

### Conjugation of Ac-C-TEG-Aβ(21-37) peptide to chloroacetylated oligotuftsin derivative carrier Ac-T20-GGGGG(ClAc)₂

Chloroacetylated oligotuftsin derivative carrier Ac-[TKPK(CIAc-GGGGG)G][TKPKG][TKPK(ClAc-GGGGG)G][TKPKG]-NH₂ (Ac-T20-GGGGG(CIAc)₂) was dissolved in 1 mg/mL sodium carbonate (aqueous solution) at a concentration of 1 mg/mL and then the Ac-C-TEG-Aβ21-37 peptide in solid form was added in only one portion to the carrier solution (3-fold excess corresponding to the CIAc-groups). The conjugation was carried out for 16 hrs at RT. The (Ac-C-TEG-Aβ21-37)₂ disulfide dimer formed during the conjugation reaction was reduced to the monomeric Ac-C-TEG-Aβ21-37 by the addition of DTT (reaction carried out for 1 h at RT) and then the reaction mixture was acidified with TFA to pH 3 and the bioconjugate was separated by preparative RP-HPLC on a C₄ column.

### Example 2

### Immunization of rats with Aβ(21-37) bioconjugates and affinity of antisera

### Protocol 1

### a) Antibody production

Male Wistar rats can be immunized with the Aβ(21-37) T-20 bioconjugates comprising 1 or 4 copies of the plaque-specific N-terminal Aβ(21-37) epitope and an isolated T-epitope, appropriate to the mouse strain being used for immunization.

Bioconjugates emulsified in Complete Freund's Adjuvant are administered by injecting 100 µg dose of peptide/animal s.c. in the foot-pad and tail. Three animals are used in the immunized and control groups. Immunization intervals were a) second injection after four weeks, b) then further boosting two times at two-week intervals, using 50 µg peptide/animal at the second, third, and fourth injection. Blood samples are taken after the fourth immunization, and analyzed by ELISA.

Binding affinities of Aβ(21-37)-bioconjugates are determined by ELISA as described below.

### b) Affinity characterization by ELISA

ELISA affinity binding studies are performed as follows:
1) coat the 96-well ELISA plates with 100 µL/well of conjugate solution (c=1 µM in PBS, pH 7.4); overnight incubation at room temperature.
2) wash the plates four times with 200 µL/well of washing buffer (PBS-T: 0.05% Tween-20 in PBS buffer, pH 7.4);
3) block with 200 µL/well of blocking buffer (5% BSA, 0.1% Tween-20 in PBS), for 2 h at room temperature;
4) wash once with 200 µL/well PBS-T;
5) 100 µL/well of rat or mouse serum is added at an initial dilution of 1:100, then diluted 3 fold serially in sample dilution buffer (5% BSA, 0.1% Tween-20 in PBS;
6) wash six times with 200 µL/well PBS-T;
7) 100 µL/well of goat anti-mouse or goat anti-rat IgG conjugated with horseradish peroxidase diluted 1:5000 in blocking buffer is added to the plates and incubated for 1 h at room temperature;
8) wash three times with 200 µL/well of BPS-T and once with 200 µL/well of 0.05 M sodium phosphate-citrate buffer, pH=5;
9) 100 µL of *o*-phenylenediamine dihydrochloride (OPD) in substrate buffer (phosphate-citrate) at c=1 mg/mL with 2 µL if 30% hydrogen-peroxide per 10 mL of substrate buffer is added;
10) The absorbance at I=450 nm is measured on a Wallac 1420 Victor2 ELISA Reader.

### Protocol 2

6 Balb/c mice can be immunized with synthetic bioconjugate (Ac-Tcell-T20(Ac-C-TEG-Abeta21-37)3/4. Blood is collected from all mice prior to the primary immunization (prebleeds). Primary immunization is completed IP in CFA (20µg protein in 100µl). Two booster immunizations are given IP in IFA at 2 weeks and 4 weeks post the primary immunization (20µg protein in 100µl). Blood samples are taken 1 week following the 3rd immunization. Serum from prebleeds and post-3rd dose bleeds are tested by ELISA against the following antigens:
■ Ac-T20 carrier protein
■ Aprotinin (irrelevant protein)
■ Bioconjugate #12
■ Abeta 1-40 monomer
■ Abeta 1-40 cis(dimer)

### Anti-Beta Amyloid Antibody ELISA Protocol

Ac-T20 carrier, bioconjugate (Ac-Tcell-T20(Ac-C-TEG-Abeta21-37)3/4, Aβ(1-40) monomer, Aβ(1-40) cis-dimer, and cAprotinin (Sigma) are immobilized at 1µg/ml in PBS (50 µl per well) on a Nunc Maxisorb 96-well plate overnight at 4°C. Wells are washed once with 350 µl Wash Buffer (1X PBS, 0.05% (v/v) Tween-20 (Sigma)). Wells are then blocked with Blocking Buffer (2% (w/v) Difco Skim milk (BD) in PBS, 50-150 µl per well) for 2 hours at room-temperature and washed once with 350 µl Wash Buffer. Serum from prebleeds and post-3rd dose bleeds are serially diluted from 1/50 to 1/3200 in Antibody Buffer (1% (w/v) Bovine Serum Albumin (Sigma) in PBS, 0.05% Tween-20 (Sigma). The control antibody 6E10 mAb (Sigma) is analyzed at a starting concentration of 1 µg/ml. Diluted serum and control antibody samples (100 µl) are transferred to Nunc Maxisorb plates and incubated for 2-3 hours at room-temperature. Wells are washed three times with 350 µl Wash Buffer rapidly.
The secondary antibody sheep anti-mouse IgG-HRP (Chemicon) is added at 1:1000 in Antibody Buffer (50 µl per well) and incubated for 30 minutes at room-temperature. Wells are then washed three times with 350 µl Wash Buffer and the plates developed with TMB Substrate (Millipore, Australia) (50 µl per well) for 5 minutes. The reaction is stopped with 2M phosphoric acid (25 µl per well) and the plates were read at 450nm, 0.1 seconds in Wallac Victor 2 plate reader.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

It is to be understood that various alterations or additions may be made to the parts previously described without departing from the spirit or ambit of the present invention.

### List of Sequences:

- SEQ ID NO. 1: DAEFRHDSGYEVHHQKLVFFAEDVGSNKGAIIGLMVGGVVIA
- SEQ ID NO. 2: DAEFRHDSGYEVHHQKLVFFAEDVGSNKGAIIGLMVGGVV
- SEQ ID NO. 3: FRHDSGY
- SEQ ID NO. 4: AEDVGSNKGAIIGLMVG
- SEQ ID NO. 5: AIIGLMVG
- SEQ ID NO. 6: GAIIGLMVG
- SEQ ID NO. 7: KGAIIGLMVG
- SEQ ID NO. 8: NKGAIIGLMVG
- SEQ ID NO. 9: SNKGAIIGLMVG
- SEQ ID NO. 10: GSNKGAIIGLMVG
- SEQ ID NO. 11: VGSNKGAIIGLMVG
- SEQ ID NO. 12: DVGSNKGAIIGLMVG
- SEQ ID NO. 13: EDVGSNKGAIIGLMVG
- SEQ ID NO. 14: FAEDVGSNKGAIIGLMVG
- SEQ ID NO. 15: FFAEDVGSNKGAIIGLMVG
- SEQ ID NO. 16: VFFAEDVGSNKGAIIGLMVG
- SEQ ID NO. 17: LVFFAEDVGSNKGAIIGLMVG
- SEQ ID NO. 18: KLVFFAEDVGSNKGAIIGLMVG
- SEQ ID NO. 19: QKLVFFAEDVGSNKGAIIGLMVG
- SEQ ID NO. 20: HQKLVFFAEDVGSNKGAIIGLMVG
- SEQ ID NO. 21: HHQKLVFFAEDVGSNKGAIIGLMVG
- SEQ ID NO. 22: VHHQKLVFFAEDVGSNKGAIIGLMVG
- SEQ ID NO. 23: EVHHQKLVFFAEDVGSNKGAIIGLMVG
- SEQ ID NO. 24: VHHQKLVFFAEDVGSNKGAIIGLMVGGVV
- SEQ ID NO. 25: DAEFRHDSGYEVHHQK
- SEQ ID NO. 26: FAEDVGSNKGA
- SEQ ID NO. 27: LVFFAEDVGSNK
- SEQ ID NO. 28: LVFFAEDVGSNKGAIIGLMVGGVVIA
- SEQ ID NO. 29: EKKIAKMEKASSVFNVVNS
- SEQ ID NO. 30: IEQYLKKIKNSISTEWSPCS
- SEQ ID NO. 31: QYIKANSKFIGITEL
- SEQ ID NO. 32: FNNFTVSFWLRVPKVSASHLE
- SEQ ID NO. 33: NSVDDALINSTKIYSYFPSV
- SEQ ID NO. 34: PGINGKAIHLVNNESSE
- SEQ ID NO. 35: QYIKANSKFIGITE
- SEQ ID NO. 36: SGPLKAEIAQRLE
- SEQ ID NO. 37: PKYVKQNTLKLAT
- SEQ ID NO. 38: LSEIKGVIVHRLEGV
- SEQ ID NO. 39: FFLLTRILTIPQSLD
- SEQ ID NO. 40: NVPDPQVGITTMRDLKG
- SEQ ID NO. 41: MSRIAAGDLESSVDDPRSEEDRR
- SEQ ID NO. 42: DRAHYNI

## Claims

1. A bioconjugate comprising a scaffold and an antigen associated therewith, wherein the antigen is a peptide comprising an amino acid sequence from a β-amyloid peptide consisting of EVHHQKLVFFAEDVGSNKGAIIGLMVG [SEQ ID NO. 23] or a fragment thereof comprising AIIGLMVG [SEQ ID NO. 5].

2. The bioconjugate of claim 1, wherein the antigen is present as a homo- or hetero-oligomer.

3. The bioconjugate of claim 2, wherein the antigen is a homo- or hetero-dimer.

4. The bioconjugate according to any one of claims 1 to 3, wherein the antigen is covalently linked to the scaffold.

5. The bioconjugate according to any one of claims 1 to 4, wherein the scaffold is selected from the group consisting of an oligotuftsin derivative, a sequential oligopeptide carrier (SOC), a multiple antigenic protein (MAP), a branched peptide or non-peptide, virus like particles and liposomes.

6. The bioconjugate according to claim 5, wherein the bioconjugate has the formula: 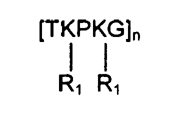 wherein T is threonine, K is lysine, P is proline, G is glycine and R₁ is a residue representing either H or -X-Z wherein X is a linker and Z is the dimer of the peptide with the proviso that at least one R₁ is not H.

7. The bioconjugate according to claim 6, wherein n is 2-4.

8. The bioconjugate according to any one of claims 1 to 7, wherein the antigen is associated with the scaffold by a linker.

9. The bioconjugate of claim 8, wherein the linker is selected from the group consisting of a thioether linkage, a polyglycine residue having 4-12 glycine residues, a short peptide having the amino acid sequence GFLG and an oligoethylene glycol.

10. A pharmaceutical composition comprising the bioconjugate according to any of claims 1 to 9 and a T-helper cell epitope.

11. The pharmaceutical composition of claim 10, wherein the T-helper cell epitope is an isolated peptide.

12. The pharmaceutical composition of claim 11, wherein the T-helper cell epitope is covalently linked to the scaffold.

13. The pharmaceutical composition of claim 10, wherein the T-helper cell epitope is contained within a polypeptide.

14. The pharmaceutical composition of claim 13, wherein the T-helper cell epitope is associated with the bioconjugate of any one of claims 1 to 9.

15. A vaccine comprising the pharmaceutical composition of any one of claims 10 to 14 and an adjuvant.

16. Use of the vaccine of claim 15 in the prevention or treatment of Alzheimer's disease.

17. A diagnostic test for *in vitro* diagnosis of Alzheimer's disease comprising the use of a bioconjugate according to any of claims 1-9.

18. A method for the *in vitro* diagnosis of Alzheimer's disease in a patient comprising contacting a sample obtained from said patient with the bioconjugate according to any one of claims 1 to 9 and detecting the binding of specific antibodies to said bioconjugate.


SEQUENCE LISTING

<110>  Universität Konstanz

<120>  Bioconjugates comprising Ab-autoantibody specific epitopes for
       active Immunotherapie and diagnosis of Alzheimer´disease

<130>  110708

<160>  42

<170>  PatentIn version 3.5

<210>  1
<211>  42
<212>  PRT
<213>  Artificial Sequence

<220>
<223>  peptide

<400>  1

<210>  2
<211>  40
<212>  PRT
<213>  Artificial Sequence

<220>
<223>  peptid

<400>  2

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  3
<211>  7
<212>  PRT
<213>  Artificial Sequence

<220>
<223>  peptid

<400>  3

Phe Arg His Asp Ser Gly Tyr

1               5


<210>  4
<211>  17
<212>  PRT
<213>  Artificial Sequence

<220>
<223>  peptid

<400>  4

Ala Glu Asp Val Gly Ser Asn Lys Gly Ala Ile Ile Gly Leu Met Val
1               5                   10                  15


Gly



<210>  5
<211>  8
<212>  PRT
<213>  Artificial Sequence

<220>
<223>  peptid

<400>  5

Ala Ile Ile Gly Leu Met Val Gly
1               5


<210>  6
<211>  9
<212>  PRT
<213>  Artificial Sequence

<220>
<223>  peptid

<400>  6

Gly Ala Ile Ile Gly Leu Met Val Gly
1               5


<210>  7
<211>  10
<212>  PRT
<213>  Artificial Sequence

<220>
<223>  peptid

<400>  7

Lys Gly Ala Ile Ile Gly Leu Met Val Gly
1               5                   10


<210>  8
<211>  11
<212>  PRT
<213>  Artificial Sequence

<220>
<223>  peptid

<400>  8

Asn Lys Gly Ala Ile Ile Gly Leu Met Val Gly
1               5                   10


<210>  9
<211>  12
<212>  PRT
<213>  Artificial Sequence

<220>
<223>  peptid

<400>  9

Ser Asn Lys Gly Ala Ile Ile Gly Leu Met Val Gly
1               5                   10


<210>  10
<211>  13
<212>  PRT
<213>  Artificial Sequence

<220>
<223>  peptid

<400>  10

Gly Ser Asn Lys Gly Ala Ile Ile Gly Leu Met Val Gly
1               5                   10


<210>  11
<211>  14
<212>  PRT
<213>  Artificial Sequence

<220>
<223>  peptid

<400>  11

Val Gly Ser Asn Lys Gly Ala Ile Ile Gly Leu Met Val Gly
1               5                   10


<210>  12
<211>  15
<212>  PRT
<213>  Artificial Sequence

<220>
<223>  peptid

<400>  12

Asp Val Gly Ser Asn Lys Gly Ala Ile Ile Gly Leu Met Val Gly
1               5                   10                  15


<210>  13
<211>  16
<212>  PRT

<213>  Artificial Sequence

<220>
<223>  peptid

<400>  13

Glu Asp Val Gly Ser Asn Lys Gly Ala Ile Ile Gly Leu Met Val Gly
1               5                   10                  15


<210>  14
<211>  18
<212>  PRT
<213>  Artificial Sequence

<220>
<223>  peptid

<400>  14

Phe Ala Glu Asp Val Gly Ser Asn Lys Gly Ala Ile Ile Gly Leu Met
1               5                   10                  15


Val Gly



<210>  15
<211>  19
<212>  PRT
<213>  Artificial Sequence

<220>
<223>  peptid

<400>  15

Phe Phe Ala Glu Asp Val Gly Ser Asn Lys Gly Ala Ile Ile Gly Leu
1               5                   10                  15


Met Val Gly



<210>  16
<211>  20
<212>  PRT
<213>  Artificial Sequence

<220>
<223>  peptid

<400>  16

Val Phe Phe Ala Glu Asp Val Gly Ser Asn Lys Gly Ala Ile Ile Gly
1               5                   10                  15


Leu Met Val Gly
            20


<210>  17
<211>  21
<212>  PRT

<213>  Artificial Sequence

<220>
<223>  peptid

<400>  17

Leu Val Phe Phe Ala Glu Asp Val Gly Ser Asn Lys Gly Ala Ile Ile
1               5                   10                  15

Gly Leu Met Val Gly
            20

<210>  18
<211>  22
<212>  PRT
<213>  Artificial Sequence

<220>
<223>  peptid

<400>  18

Lys Leu Val Phe Phe Ala Glu Asp Val Gly Ser Asn Lys Gly Ala Ile
1               5                   10                  15

Ile Gly Leu Met Val Gly
            20

<210>  19
<211>  23
<212>  PRT
<213>  Artificial Sequence

<220>
<223>  peptid

<400>  19

Gln Lys Leu Val Phe Phe Ala Glu Asp Val Gly Ser Asn Lys Gly Ala
1               5                   10                  15

Ile Ile Gly Leu Met Val Gly
            20

<210>  20
<211>  24
<212>  PRT
<213>  Artificial Sequence

<220>
<223>  peptid

<400>  20

His Gln Lys Leu Val Phe Phe Ala Glu Asp Val Gly Ser Asn Lys Gly
1               5                   10                  15

Ala Ile Ile Gly Leu Met Val Gly
            20

<210>  21
<211>  25
<212>  PRT
<213>  Artificial Sequence

<220>
<223>  peptid

<400>  21

His His Gln Lys Leu Val Phe Phe Ala Glu Asp Val Gly Ser Asn Lys
1               5                   10                  15

Gly Ala Ile Ile Gly Leu Met Val Gly
            20                  25

<210>  22
<211>  26
<212>  PRT
<213>  Artificial Sequence

<220>
<223>  peptid

<400>  22

Val His His Gln Lys Leu Val Phe Phe Ala Glu Asp Val Gly Ser Asn
1               5                   10                  15

Lys Gly Ala Ile Ile Gly Leu Met Val Gly
            20                  25

<210>  23
<211>  27
<212>  PRT
<213>  Artificial Sequence

<220>
<223>  peptid

<400>  23

Glu Val His His Gln Lys Leu Val Phe Phe Ala Glu Asp Val Gly Ser
1               5                   10                  15

Asn Lys Gly Ala Ile Ile Gly Leu Met Val Gly
            20                  25

<210>  24
<211>  29
<212>  PRT
<213>  Artificial Sequence

<220>
<223>  peptid

<400>  24

Val His His Gln Lys Leu Val Phe Phe Ala Glu Asp Val Gly Ser Asn
1               5                   10                  15

Lys Gly Ala Ile Ile Gly Leu Met Val Gly Gly Val Val
            20                  25

<210>  25
<211>  16
<212>  PRT
<213>  Artificial Sequence

<220>
<223>  peptid

<400>  25

Asp Ala Glu Phe Arg His Asp Ser Gly Tyr Glu Val His His Gln Lys
1               5                   10                  15

<210>  26
<211>  11
<212>  PRT
<213>  Artificial Sequence

<220>
<223>  peptid

<400>  26

Phe Ala Glu Asp Val Gly Ser Asn Lys Gly Ala
1               5                   10

<210>  27
<211>  12
<212>  PRT
<213>  Artificial Sequence

<220>
<223>  peptid

<400>  27

Leu Val Phe Phe Ala Glu Asp Val Gly Ser Asn Lys
1               5                   10

<210>  28
<211>  26
<212>  PRT
<213>  Artificial Sequence

<220>
<223>  peptid

<400>  28

Leu Val Phe Phe Ala Glu Asp Val Gly Ser Asn Lys Gly Ala Ile Ile
1               5                   10                  15

Gly Leu Met Val Gly Gly Val Val Ile Ala
            20                  25

<210>  29
<211>  19
<212>  PRT

<213>  Artificial Sequence

<220>
<223>  peptid

<400>  29

Glu Lys Lys Ile Ala Lys Met Glu Lys Ala Ser Ser Val Phe Asn Val
1               5                   10                  15

Val Asn Ser

<210>  30
<211>  20
<212>  PRT
<213>  Artificial Sequence

<220>
<223>  peptid

<400>  30

Ile Glu Gln Tyr Leu Lys Lys Ile Lys Asn Ser Ile Ser Thr Glu Trp
1               5                   10                  15

Ser Pro Cys Ser
            20

<210>  31
<211>  15
<212>  PRT
<213>  Artificial Sequence

<220>
<223>  peptid

<400>  31

Gln Tyr Ile Lys Ala Asn Ser Lys Phe Ile Gly Ile Thr Glu Leu
1               5                   10                  15

<210>  32
<211>  21
<212>  PRT
<213>  Artificial Sequence

<220>
<223>  peptid

<400>  32

Phe Asn Asn Phe Thr Val Ser Phe Trp Leu Arg Val Pro Lys Val Ser
1               5                   10                  15

Ala Ser His Leu Glu
            20

<210>  33
<211>  20
<212>  PRT

<213>  Artificial Sequence

<220>
<223>  peptid

<400>  33

Asn Ser Val Asp Asp Ala Leu Ile Asn Ser Thr Lys Ile Tyr Ser Tyr
1               5                   10                  15

Phe Pro Ser Val
            20

<210>  34
<211>  17
<212>  PRT
<213>  Artificial Sequence

<220>
<223>  peptid

<400>  34

Pro Gly Ile Asn Gly Lys Ala Ile His Leu Val Asn Asn Glu Ser Ser
1               5                   10                  15

Glu

<210>  35
<211>  14
<212>  PRT
<213>  Artificial Sequence

<220>
<223>  peptid

<400>  35

Gln Tyr Ile Lys Ala Asn Ser Lys Phe Ile Gly Ile Thr Glu
1               5                   10

<210>  36
<211>  13
<212>  PRT
<213>  Artificial Sequence

<220>
<223>  peptid

<400>  36

Ser Gly Pro Leu Lys Ala Glu Ile Ala Gln Arg Leu Glu
1               5                   10

<210>  37
<211>  13
<212>  PRT
<213>  Artificial Sequence

<220>
<223>  peptid

<400>  37

Ser Gly Pro Leu Lys Ala Glu Ile Ala Gln Arg Leu Glu
1               5                   10


<210>  38
<211>  15
<212>  PRT
<213>  Artificial Sequence

<220>
<223>  peptid

<400>  38

Leu Ser Glu Ile Lys Gly Val Ile Val His Arg Leu Glu Gly Val
1               5                   10                  15


<210>  39
<211>  15
<212>  PRT
<213>  Artificial Sequence

<220>
<223>  peptid

<400>  39

Phe Phe Leu Leu Thr Arg Ile Leu Thr Ile Pro Gln Ser Leu Asp
1               5                   10                  15


<210>  40
<211>  17
<212>  PRT
<213>  Artificial Sequence

<220>
<223>  peptid

<400>  40

Asn Val Pro Asp Pro Gln Val Gly Ile Thr Thr Met Arg Asp Leu Lys
1               5                   10                  15


Gly


<210>  41
<211>  23
<212>  PRT
<213>  Artificial Sequence

<220>
<223>  peptid

<400>  41

Met Ser Arg Ile Ala Ala Gly Asp Leu Glu Ser Ser Val Asp Asp Pro
1               5                   10                  15


Arg Ser Glu Glu Asp Arg Arg

            20

<210>  42
<211>  7
<212>  PRT
<213>  Artificial Sequence

<220>
<223>  peptid

<400>  42

Asp Arg Ala His Tyr Asn Ile
1               5